(19) **Europäisches Patentamt / European Patent Office / Office européen des brevets**

(11) **EP 4 260 321 B1**

(12) **EUROPEAN PATENT SPECIFICATION**

(45) Date of publication and mention of the grant of the patent:
**11.06.2025 Bulletin 2025/24**

(21) Application number: **21902906.3**

(22) Date of filing: **09.12.2021**

(51) International Patent Classification (IPC):
*G16B 5/20* (2019.01)    *G16B 5/00* (2019.01)
*G16H 50/30* (2018.01)    *G16B 30/00* (2019.01)
*G16H 20/10* (2018.01)    *C12Q 1/6869* (2018.01)
*G16B 20/00* (2019.01)    *G16B 20/40* (2019.01)
*G16B 10/00* (2019.01)    *C12Q 1/689* (2018.01)
*G16B 30/10* (2019.01)

(52) Cooperative Patent Classification (CPC):
**G16B 10/00; G16B 20/40; G16B 30/00; G16B 30/10**

(86) International application number:
**PCT/IN2021/051159**

(87) International publication number:
**WO 2022/123606 (16.06.2022 Gazette 2022/24)**

(54) **METHOD AND SYSTEM FOR SIMULTANEOUS INTERPRETATION OF TAXONOMIC DISTRIBUTION AND REPLICATION RATES OF MICROBIAL COMMUNITIES**

VERFAHREN UND SYSTEM ZUR SIMULTANEN INTERPRETATION TAXONOMISCHER VERTEILUNG UND REPLIKATIONSRATEN MIKROBIELLER GEMEINSCHAFTEN

PROCÉDÉ ET SYSTÈME D'INTERPRÉTATION SIMULTANÉE DE DISTRIBUTION TAXONOMIQUE ET DE TAUX DE RÉPLICATION DE COMMUNAUTÉS MICROBIENNES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **09.12.2020 IN 202021053578**

(43) Date of publication of application:
**18.10.2023 Bulletin 2023/42**

(73) Proprietor: **Tata Consultancy Services Limited Maharashtra (IN)**

(72) Inventors:
• DUTTA, Anirban
  Pune 411013 (IN)
• PINNA, Nishal Kumar
  Pune 411013 (IN)
• BHAR, Subhrajit
  Pune 411013 (IN)
• BOSE, Tungadri
  Pune 411013 (IN)
• MANDE, Sharmila Shekhar
  Pune 411013 (IN)

(74) Representative: **Goddar, Heinz J.**
**Boehmert & Boehmert**
**Anwaltspartnerschaft mbB**
**Pettenkoferstrasse 22**
**80336 München (DE)**

(56) References cited:
JP-A- 2017 533 723    JP-A- 2017 533 723
US-A1- 2020 115 766    US-A1- 2020 115 766

• BROWN CHRISTOPHER T ET AL: "Measurement of bacterial replication rates in microbial communities", NATURE BIOTECHNOLOGY, vol. 34, no. 12, 7 November 2016 (2016-11-07), New York, pages 1256 - 1263, XP055944071, ISSN: 1087-0156, Retrieved from the Internet <URL:http://www.nature.com/articles/nbt.3704> DOI: 10.1038/nbt.3704

EP 4 260 321 B1

• T. KOREM ET AL: "Growth dynamics of gut microbiota in health and disease inferred from single metagenomic samples", SCIENCE, vol. 349, no. 6252, 4 September 2015 (2015-09-04), US, pages 1101 - 1106, XP055557436, ISSN: 0036-8075, DOI: 10.1126/science.aac4812

• BROWN, CT ET AL.: "Measurement of bacterial replication rates in microbial communities", NAT BIOTECHNOL, vol. 34, no. 12, December 2016 (2016-12-01), pages 1256 - 1263, XP055944071, DOI: 10.1038/nbt.3704

**Description**

TECHNICAL FIELD

The disclosure herein generally relates to the field of taxonomic profiling of microbial organisms such as bacteria, and, more particularly, to system and method for simultaneous interpretation of taxonomic distribution and replication rates of microbes constituting microbial communities.

BACKGROUND

Next generation sequencing (NGS) technologies have enabled probing of microbial diversity in different environmental niches with unprecedented sequencing depth wherein these techniques have immense implications in understanding human health and wellness on one hand and in applications related to environmental impact and agricultural economic assessment on the other.

Two widely used microbiome sequencing protocols include (A) Amplicon sequencing of taxonomic phylogenetic marker genes (such as, 16S rRNA gene) and (B) Whole genome shotgun (WGS) sequencing-based metagenomics. Amplicon sequencing of 16S rRNA genes is a standard protocol for taxonomic characterization of bacteria. The 16S protocols allow reconstruction of the taxonomic distribution of a bacterial ecosystem through amplification, sequencing and abundance calculation of the taxonomic/phylogenetic marker gene encoding 16S rRNA. Further, such amplicon sequencing based protocol can be extended to other taxonomic/ phylogenetic marker genes (such as housekeeping genes i.e., cpn60, gyrB, rpoB, tufA, and the like.) as well to the same effect. On the other hand, WGS (Whole genome sequencing) methods break-up the entire DNA (Deoxyribonucleic Acid) content sampled from the studied bacterial ecosystem, use random PCR (Polymerase chain reaction) amplification as necessary, and subsequently sequence and compute abundance of the genomic fragments obtained. Further, in this case, mapping back (taxonomic binning or taxonomic classification) of the genomic fragments to the bacteria of origin is more difficult than the 16S protocols and other similar amplicon sequencing based protocols, provided - (i) these fragments are randomly distributed all over the source genomes, (ii) at times there are low complexity regions on the source genome, and (iii) more than often these fragments do not contain good taxonomic signatures (or information). However, once the binning process has been efficiently performed, WGS methods not only can provide an overview of the taxonomic distribution of the ecosystem, but also the potential functional contents therein, since the sequenced fragments have their origin in multiple genes and not only the taxonomic phylogenetic marker genes as in case of 16S protocols. Despite the additional information available from WGS studies, it is manifold costlier than the (16S) amplicon sequencing method, and the latter remains the method of choice in studies aimed at understanding bacterial diversity of an ecosystem. In addition, methods to estimate the potential functional components of a microbiome from 16S rRNA information also exists.

The taxonomic proportions that are conventionally measured may however be incorrect in the absence of a reliable way to confirm how viable are the cells belonging to different taxonomic groups as the bacterial DNA that can be collected from microbiome samples can come from both live and dead cells. Further no direct inference can be derived related to bacterial interactions from a single snapshot of taxonomic distribution. Such information pertaining to replication rates and co-growth of bacterial species would normally need at least a second sampling (or more) to generate a longitudinal data and comparison of bacterial abundance at subsequent timepoints. One of the recent studies has indicated that the WGS method can help elucidating this additional dimension of information pertaining to bacterial populations. The distribution of sequence fragments (or reads) mapped onto the chromosome of a potential source organism (which can be obtained after the read mapping/ binning step) can be utilized to arrive at the replication rate of the organism (Korem et al, Growth dynamics of gut microbiota in health and disease inferred from single metagenomic samples - doi: 10.1126/science.aac4812., Brown et al., Measurement of bacterial replication rates in microbial communities -https://doi.org/10.1038/nbt.3704). The possibility of deriving the replication rate of bacterial species from WGS data makes the necessity of subsequent sampling irrelevant. Additionally, if multi-batch sequencing, which is needed for sampling at multiple timepoints, can be avoided, concerns related to batch effects will not arise.

However, as mentioned before, WGS sequencing method is manifold costlier and resource consuming than amplicon sequencing based microbiome study protocols. This makes the adoption of WGS protocol financially infeasible to be adopted for study designs involving larger sample sizes.

SUMMARY

The present invention is defined in the independent claims. The dependent claims define embodiments of the present invention.

Embodiments of the present disclosure present technological improvements as solutions to one or more of the above-mentioned technical problems recognized by the inventors in conventional systems. For example, in one embodiment, a method for simultaneous interpretation of taxonomic distribution and replication rates (SITAR) of microbial communities is provided. The method includes collecting a microbiome sample from a given environment; extracting bacterial genomic DNA (Deoxyribonucleic Acid) from a plurality of bacterial organisms constituting the collected microbiome sample; performing an amplicon sequencing by a PCR (Polymerase chain reaction) amplification module and a sequencer, on the extracted bacterial genomic DNA comprising at least one of (i) targeting two or more phylogenetic marker genes and (ii) selecting a portion from each of the two or more phylogenetic marker genes to obtain a plurality of DNA sequence fragment reads, wherein the two or more phylogenetic marker genes are found in genomes of organisms, and which are used for identification of the taxonomic lineage of the organism; mapping, by a processor, the plurality of the DNA sequence fragment reads to a precomputed reference sequence database of a plurality of available completely sequenced bacterial genomes; identifying, by the processor, a plurality of bacterial organisms in the collected microbiome sample and assigning a taxonomic classification to the identified plurality of bacterial organisms based on the mapping of the plurality of the DNA sequence fragment reads to the precomputed reference sequence database; measuring, by the processor, read coverage at the genomic locations of the two or more phylogenetic marker genes for the plurality of identified bacterial organisms based on the mapping of the plurality of the DNA sequence fragment reads to the precomputed reference sequence database, and wherein the measured read coverage is used for the interpretation of the taxonomic distribution of the plurality of bacterial organisms identified from the collected microbiome sample; fitting, by the processor, a linear function of the form $y = mx + c$ for each of the plurality of bacterial organisms by using the measured read coverage and the information of the genomic locations corresponding to the two or more phylogenetic marker genes with respect to an origin of replication (ori) and a terminus of replication (ter) specific to each of the plurality of bacterial organisms identified from the collected microbiome sample; obtaining, by the processor, a slope *(m)* from the fitted linear function of the form $y = mx + c$ for each of the plurality of bacterial organisms identified from the collected microbiome sample; estimating, by the processor, for each of the plurality of bacterial organisms identified from the collected microbiome sample an expected read coverage at the origin of replication $y_{orl}$ using the slope $(m)$ obtained and the value c from the fitted linear function; estimating, by the processor, for each of the plurality of bacterial organisms identified from the collected microbiome sample an expected read coverage at the terminus of replication $y_{ter}$ using the slope *(m)* obtained and the value c from the fitted linear function; and interpreting, by a processor, for each of the plurality of bacterial organisms identified from the collected microbiome sample a replication rate using at least one of (i) slopes *(m)* and (ii) ratio of $\frac{y_{ori}}{y_{ter}}$ .

In another aspect, there is provided a system for simultaneous interpretation of taxonomic distribution and replication rates (SITAR) of microbial communities is provided. The system comprises a sample collection module for collecting the microbiome sample from a given environment; a DNA extraction module for extracting bacterial genomic DNA from a plurality of bacterial organisms constituting the collected microbiome sample; a PCR amplification module and a sequencer for performing an amplicon sequencing, on the extracted bacterial genomic DNA comprising at least one of (i) targeting two or more phylogenetic marker genes and (ii) selecting a portion from each of the two or more phylogenetic marker genes to obtain a plurality of DNA sequence fragment reads, wherein the phylogenetic marker genes are found in genomes of organisms, and which are used for identification of the taxonomic lineage of the organism; a memory and a processor in communication with the memory, wherein the processor configured to perform the steps of: mapping, the plurality of the DNA sequence fragment reads to a precomputed reference sequence database of a plurality of available completely sequenced bacterial genomes; identifying a plurality of bacterial organisms in the collected microbiome sample and assigning a taxonomic classification to the identified plurality of bacterial organisms based on the mapping of the plurality of the DNA sequence fragment reads to the precomputed reference sequence database; measuring read coverage at the genomic locations of the two or more phylogenetic marker genes for the plurality of identified bacterial organisms based on the mapping of the plurality of the DNA sequence fragment reads to the precomputed reference sequence database, and wherein the measured read coverage is used for the interpretation of the taxonomic distribution of the plurality of bacterial organisms identified from the collected microbiome sample; fitting a linear function of the form $y = mx + c$ for each of the plurality of bacterial organisms by using the measured read coverage and the information of the genomic locations corresponding to the two or more phylogenetic marker genes with respect to an origin of replication (ori) and a terminus of replication (ter) specific to each of the plurality of bacterial organisms identified from the collected microbiome sample; obtaining a slope $(m)$ from the fitted linear function of the form $y = mx + c$ for each of the plurality of bacterial organisms identified from the collected microbiome sample; estimating for each of the plurality of bacterial organisms identified from the collected microbiome sample an expected read coverage at the origin of replication $y_{orl}$ using the slope $(m)$ obtained and the value c from the fitted linear function; estimating for each of the plurality of bacterial organisms identified from the collected microbiome sample an expected read coverage at the terminus of replication $y_{ter}$ using the slope *(m)* obtained and the value c from the fitted linear function; and interpreting for each of the plurality of bacterial organisms identified from the collected microbiome sample a replication rate using at least one of (i) slopes *(m)*

and (ii) ratio of $\dfrac{y_{ori}}{y_{ter}}$ .

In yet another aspect, there are provided one or more non-transitory machine-readable information storage mediums comprising one or more instructions which when executed by a processor cause collecting a microbiome sample from a given environment; extracting bacterial genomic DNA (Deoxyribonucleic Acid) from a plurality of bacterial organisms constituting the collected microbiome sample; performing an amplicon sequencing by a PCR (Polymerase chain reaction) amplification module and a sequencer, on the extracted bacterial genomic DNA comprising at least one of (i) targeting two or more phylogenetic marker genes and (ii) selecting a portion from each of the two or more phylogenetic marker genes to obtain a plurality of DNA sequence fragment reads, wherein the two or more phylogenetic marker genes are found in genomes of organisms, and which are used for identification of the taxonomic lineage of the organism; mapping, by a processor, the plurality of the DNA sequence fragment reads to a precomputed reference sequence database of a plurality of available completely sequenced bacterial genomes; identifying, by the processor, a plurality of bacterial organisms in the collected microbiome sample and assigning a taxonomic classification to the identified plurality of bacterial organisms based on the mapping of the plurality of the DNA sequence fragment reads to the precomputed reference sequence database; measuring, by the processor, read coverage at the genomic locations of the two or more phylogenetic marker genes for the plurality of identified bacterial organisms based on the mapping of the plurality of the DNA sequence fragment reads to the precomputed reference sequence database, and wherein the measured read coverage is used for the interpretation of the taxonomic distribution of the plurality of bacterial organisms identified from the collected microbiome sample; fitting, by the processor, a linear function of the form $y = mx + c$ for each of the plurality of bacterial organisms by using the measured read coverage and the information of the genomic locations corresponding to the two or more phylogenetic marker genes with respect to an origin of replication (ori) and a terminus of replication (ter) specific to each of the plurality of bacterial organisms identified from the collected microbiome sample; obtaining, by the processor, a slope *(m)* from the fitted linear function of the form $y = mx + c$ for each of the plurality of bacterial organisms identified from the collected microbiome sample; estimating, by the processor, for each of the plurality of bacterial organisms identified from the collected microbiome sample an expected read coverage at the origin of replication $y_{ori}$ using the slope *(m)* obtained and the value c from the fitted linear function; estimating, by the processor, for each of the plurality of bacterial organisms identified from the collected microbiome sample an expected read coverage at the terminus of replication $y_{ter}$ using the slope *(m)* obtained and the value c from the fitted linear function; and interpreting, by a processor, for each of the plurality of bacterial organisms identified from the collected microbiome sample a replication rate using at least one of (i) slopes *(m)* and (ii) ratio of $\dfrac{y_{ori}}{y_{ter}}$ .

It is to be understood that both the foregoing general description and the following detailed description are exemplary and explanatory only and are not restrictive of the invention, as claimed.

BRIEF DESCRIPTION OF THE DRAWINGS

The accompanying drawings, which are incorporated in and constitute a part of this disclosure, illustrate exemplary embodiments and, together with the description, serve to explain the disclosed principles:

FIG. 1 illustrates a block diagram of a system for simultaneous interpretation of taxonomic distribution and replication rates of microbial communities according to an embodiment of the present disclosure.

FIGS. 2A and 2B show a flowchart illustrating a method for simultaneous interpretation of taxonomic distribution and replication rates of microbial communities according to an embodiment of the present disclosure.

FIG. 3 illustrates a bacterial chromosomal replication process in accordance with some embodiments of the present disclosure.

FIG. 4 illustrates a differential coverage of a genomic loci during high-throughput sequencing experiments (e.g., WGS (Whole genome sequencing)) dependent on a distance of concerned genomic loci from an origin of replication (ori), in accordance with some embodiments of the present disclosure.

FIG. 5 illustrates a representative linear equation which can be fitted with a sequencing coverage data for two distinct genomic loci (say phylogenetic marker gene *A* and phylogenetic marker gene *B*, obtained through amplicon sequencing) and their location on the genome with respect to the origin of replication (ori), in accordance with some embodiments of the present disclosure.

FIG. 6 illustrates the Pearson's correlation coefficient between computed SITAR method's metric estimating bacterial replication rates and bPTR values obtained during the validation of the present method, in accordance with some embodiments of the present disclosure.

FIG. 7A through 7C illustrates the Pearson's correlation values computed between WGS (Whole genome sequencing) derived relative abundances and (A) 16S rRNA, (B) CPN60, (C) average of 16S rRNA and CPN60 derived relative abundances, in accordance with some embodiments of the present disclosure.

DETAILED DESCRIPTION OF EMBODIMENTS

Exemplary embodiments are described with reference to the accompanying drawings. In the figures, the left-most digit(s) of a reference number identifies the figure in which the reference number first appears. Wherever convenient, the same reference numbers are used throughout the drawings to refer to the same or like parts. While examples and features of disclosed principles are described herein, modifications, adaptations, and other implementations are possible without departing from the scope of the disclosed embodiments.

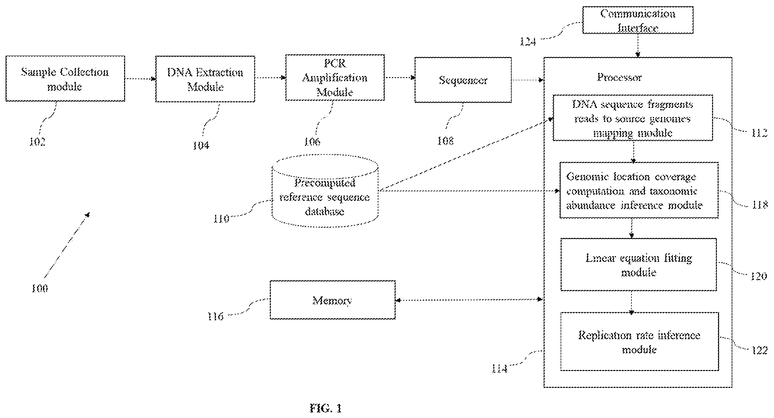

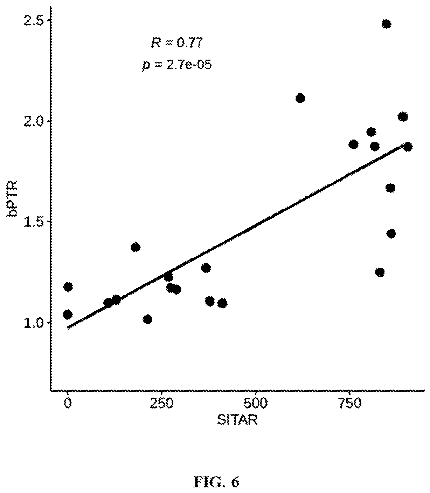

Referring now to the drawings, and more particularly to FIG. 1 through FIG. 7C, where similar reference characters denote corresponding features consistently throughout the figures, there are shown preferred embodiments and these embodiments are described in the context of the following exemplary system and/or method.

According to an embodiment of the disclosure, the system 100 comprises a sample collection module 102, a DNA extraction module 104, a PCR amplification module 106, a sequencer 108, a precomputed reference sequence database 110, a memory 116, a processor 114 and a one or more communication interfaces 124 as shown in the block diagram of Fig. 1. The processor 114 works in communication with the memory 116. The processor 114 further comprises a plurality of modules. The plurality of modules accesses the set of algorithms stored in the memory 116 to perform certain functions. The processor 114 further comprises a DNA sequence fragment reads to source genomes mapping module 112, a genomic location coverage computation and taxonomic abundance inference module 118, a linear equation fitting module 120 and a replication rate inference module 122. The one or more communication interfaces 124 can include a variety of software and hardware interfaces, for example, a web interface, a graphical user interface, and the like and can facilitate multiple communications within a wide variety of networks N/W and protocol types, including wired networks, for example, LAN, cable, etc., and wireless networks, such as WLAN, cellular, or satellite.

According to an embodiment of the disclosure, a system 100 for simultaneous interpretation of taxonomic distribution and replication rates of microbial communities is shown in block diagram of FIG. 1. The system 100 is specifically using the amplicon sequencing which is performed on the microbiome sample targeting a first phylogenetic marker gene (say A) and a second phylogenetic marker gene (say B) amplicons in parallel. In one implementation, the present disclosure ascertains genomic/chromosomal location of a 16S rRNA gene(s) and a CPN60 gene(s) for all available (sequenced) bacterial chromosomes. In alternate implementations any conserved genomic loci/genes capable of phylogenetic classification can be used as the first phylogenetic marker gene and the second phylogenetic marker gene for the amplicon sequencing. Further, the present disclosure predicts the location of the origin of replication (ori) and a terminus of replication (ter) for all collated bacterial chromosomes using state-of-art methods. The present disclosure, as a one-time step, creates location database of 16S and CPN60 genes and any other conserved genomic loci/genes capable of phylogenetic classification of microbes, that can be used as the phylogenetic marker genes for the amplicon sequencing, in terms of distance from the origin of replication (ori) and the terminus of replication (ter) (as available). The said one-time step also comprises of creating a sequence database of 16S and CPN60 genes and any other conserved genomic loci/genes capable of phylogenetic classification of microbes, that can be used as the phylogenetic marker genes for the amplicon sequencing. Taken together, the location database and the sequence database thus created constitutes the precomputed reference sequence database. In an embodiment of the present disclosure, the words "bacteria" and "microbe" can be interchangeably used. In an embodiment of the present disclosure, the words "genomic location" and "genomic loci" can be interchangeably used.

According to an embodiment of the disclosure the sample collection module 102 is configured to collect a microbiome sample from any given environment, where the microbiome sample can be collected from one of the human/animal body sites such as gut, skin, hair, nasopharynx etc. or from body fluids such as saliva, urine, blood, etc. or stool, sputum, cerumen, etc. The microbiome sample can further be collected from different parts of a plant, viz., endosphere, rhizosphere, rhizoplane, leaf, fruit, seed, etc. or from plant (and plant product) extracts. In addition, the microbiome sample can be collected from other environmental sources including sewage, bioreactor, river/ocean bed, air etc. In addition, the microbiome sample can also be collected from stored biological or organic material, including raw/processed food, food grains, natural product derived drugs and even probiotic formulations intended for therapeutic use.

According to an embodiment of the disclosure, the system 100 further comprises the DNA extraction module 104, the PCR amplification module 106, the sequencer 108 and the precomputed reference sequence database 110. The DNA extraction module 104 is configured to extract bacterial genomic DNA from the collected microbiome sample using laboratory standardized protocol. The PCR amplification module 106 is configured to perform an amplicon sequencing on the bacterial genomic DNA extracted from collected microbiome sample. In an implementation the amplicon sequencing is performed by targeting two or more phylogenetic marker genes to obtain a plurality of DNA sequence fragment reads, wherein the phylogenetic marker genes are found in genomes of organisms, and which are used for identification of the taxonomic lineage of the organism. In an alternate implementation, the amplicon sequencing is performed by selecting a portion from each of the two or more phylogenetic marker genes. Further, selecting the portion from each of the two or more phylogenetic marker genes can be based on the capability of available sequencing technology in terms of the maximum read length (maximum DNA fragment size that can be sequenced in one go). The sequencer 108 is configured to sequence the PCR amplified DNA libraries to obtain DNA sequence data constituted of a plurality of DNA sequence fragment reads corresponding to the amplicons from the first phylogenetic marker gene (say *A*) and the second phylogenetic marker gene (say *B*). The precomputed reference sequence database 110 comprises the distance of the first phylogenetic marker gene (say *A)* and the second phylogenetic marker gene (say *B*) from the origin of replication (ori) and the terminus of replication (ter), considering a linear model of the circular chromosome wherein "ori" represents start and "ter" represents end of the linear model of the chromosome. The distance of the phylogenetic marker genes was represented in a pre-computed linear scale of 0-100 with respect to the locations of the origin of replication (ori) and the terminus of replication (ter) of the respective bacterial genomes. The precomputed reference sequence database 110 further comprises of a genomic sequence database which further comprises sequences of the selected phylogenetic marker genes from all the available completely sequenced bacterial genomes.

According to an embodiment of the disclosure, the system 100 further comprises of the DNA sequence fragments reads to source genomes mapping module 112 which is configured to map back the plurality of DNA sequence fragments reads comprising the plurality of DNA sequence data to a plurality of respective locations in source genomes in the precomputed reference sequence database 110. The DNA sequence fragment reads correspond to the two or more phylogenetic marker genes. The genome location coverage computation and taxonomic abundance inference module 118 is configured to measure the read coverage data of the two or more phylogenetic marker genes for the plurality of bacterial organisms which are identified in the collected microbiome sample. The linear equation fitting module 120 is configured to fit a linear function for each bacterial organism by using the measured read coverage data and the information of the genomic locations corresponding to the two or more phylogenetic marker genes with respect to the origin of replication (ori) and the terminus of replication (ter) specific to each of the plurality of bacterial organism identified from the collected microbiome sample, and the replication rate inference module 122 is configured to interpret the replication rates of the plurality of bacterial organisms comprised in the collected microbiome sample using a plurality of slopes.

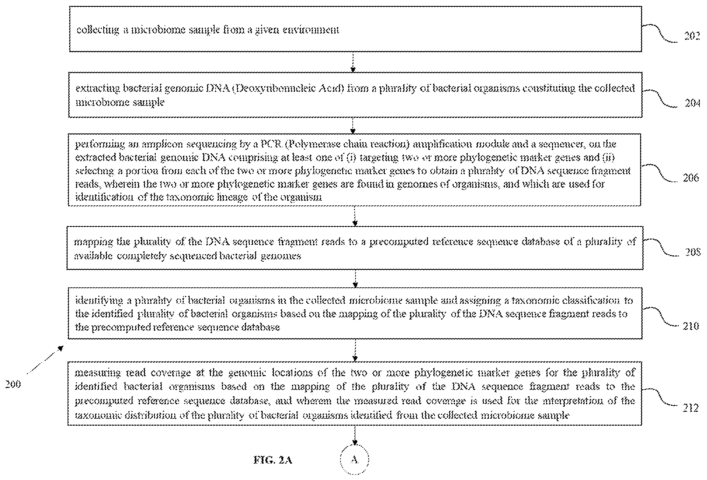

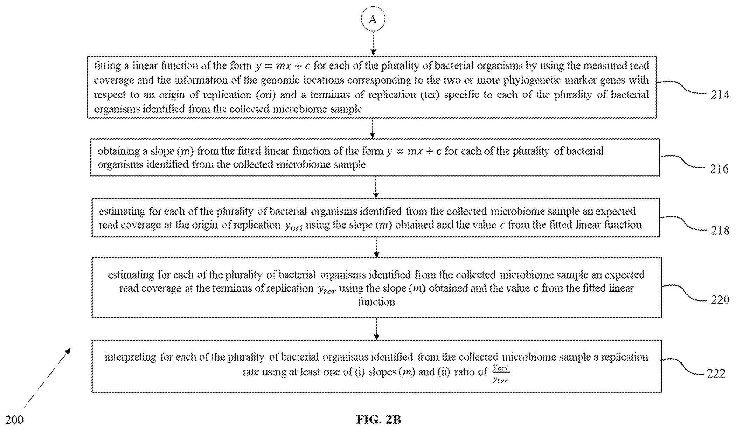

FIGS. 2A and 2B show a flowchart illustrating a method 200 for simultaneous interpretation of taxonomic distribution and replication rates of microbial community according to an embodiment of the present disclosure. Initially at step 202, a microbiome sample is collected from a given environment. At step 204, bacterial genomic DNA is extracted from a plurality of bacterial organisms constituting the collected microbiome sample. In the next step 206, an amplicon sequencing is performed on the extracted bacterial genomic DNA which comprises at least one of (i) two or more phylogenetic marker genes are targeted and (ii) a portion from each of the two or more phylogenetic marker genes are selected to obtain a plurality of DNA sequence fragment reads, wherein the phylogenetic marker genes are found in genomes of organisms, and which are used for identification of the taxonomic lineage of the organism. At step 208, the plurality of the DNA sequence fragment reads, generated post PCR amplification and sequencing of the two or more phylogenetic marker genes are mapped to a precomputed reference sequence database of a plurality of all available completely sequenced bacterial genomes.

At step 210, a plurality of bacterial organisms in the collected microbiome sample are identified and a taxonomic classification/ lineage are assigned to the identified plurality of bacterial organisms based on the mapping of the plurality of the DNA sequence fragment reads to the precomputed reference sequence database. In an implementation, the plurality of DNA sequence fragment reads correspond to the targeted two or more phylogenetic marker genes. In another implementation, the plurality of DNA sequence fragment reads correspond to the selected portion from each of the two or more phylogenetic marker genes. Taxonomic classification (or binning) is performed using state of the art algorithms and taxonomic reference databases cataloging sequences of the first phylogenetic marker gene (say *A)* and the second phylogenetic marker gene (say *B*) from known bacterial organism and further the identification can be extended to other organisms. At step 212, read coverage at the genomic locations of the two or more phylogenetic marker genes for the plurality of identified bacterial organisms is measured based on the mapping of the plurality of the DNA sequence fragment reads to the precomputed reference sequence database, and wherein the measured read coverage is used for the interpretation of the taxonomic distribution of the plurality of bacterial organisms identified from the collected microbiome sample.

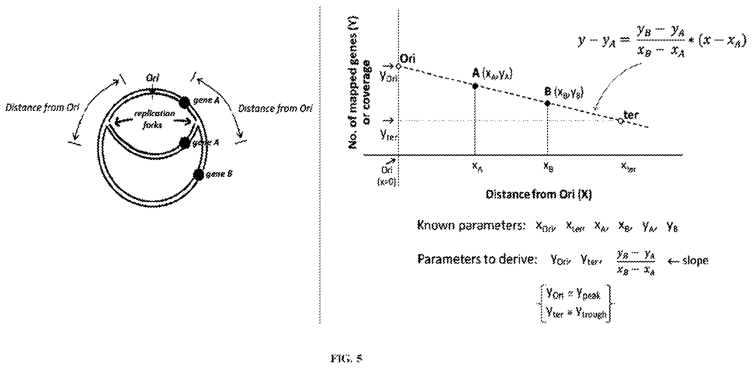

At step 214, a linear function of the form $y = mx + c$ is fitted for each of the plurality of bacterial organisms by using the measured read coverage and the information of the genomic locations corresponding to the two or more phylogenetic marker genes with respect to an origin of replication (ori) and a terminus of replication (ter) specific to each of the plurality of bacterial organisms identified from the collected microbiome sample as depicted in FIG. 5. At step 216, a slope *(m)* is obtained from the fitted linear function of the form $y = mx + c$ for each of the plurality of bacterial organisms identified from the collected microbiome sample. At step 218, for each of the plurality of bacterial organisms identified from the collected microbiome sample an expected read coverage is estimated at the origin of replication $y_{ori}$ using the slope *(m)* obtained and the value c from the fitted linear function. At step 220, for each of the plurality of bacterial organisms identified from the collected microbiome sample an expected read coverage is estimated at the terminus of replication $y_{ter}$ using the slope *(m)*

obtained and the value c from the fitted linear function. At step 222, for each of the plurality of bacterial organisms identified from the collected microbiome sample a replication rate is interpreted using at least one of (i) slopes (*m*) and (ii) ratio of $\frac{y_{ori}}{y_{ter}}$.

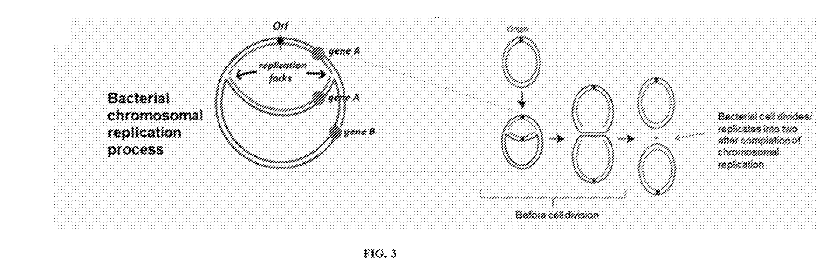

FIG. 3 illustrates a bacterial chromosomal replication process in accordance with some embodiments of the present disclosure. The premise of the present disclosure lies in the biological process of the bacterial chromosomal replication. The bacterial chromosome is circular in nature and the replication process generally starts at a particular genomic location known as the 'origin of replication' or the 'ori'. At the initiation of the replication process, the circular chromosome starts splitting into two daughter chromosomes, starting at the origin of replication (ori), wherein a 'bubble' like formation bound by two 'forks' is created around the origin of replication (ori) location as depicted in FIG. 3. As the replication process proceeds, the replication-forks spreads out further and the bubble increases in size. Eventually the forks come around and collide at a locus often referred to as the terminus of replication (ter), and two separate daughter strands are created, completing the replication process for one cycle, as depicted in the FIG. 3.

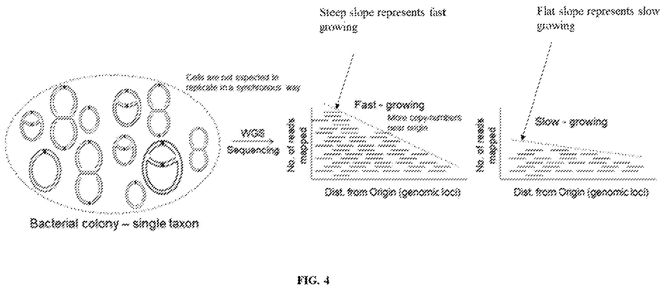

FIG. 4 illustrates a differential coverage of the genomic location during high-throughput sequencing experiments (e.g., WGS) dependent on the distance of the concerned genomic loci from the origin of replication (ori), in accordance with some embodiments of the present disclosure. In the present disclosure, at any intermediate stage of replication, any gene (or genomic loci) located close to the origin of replication (ori) are expected to have already replicated into the daughter strand, wherein the gene/genomic loci located closer to the terminus of replication (ter) may not have replicated yet. Hence, when the DNA sequencing of the genomic-loci or random fragments of the bacterial genomes from a population is performed, the copy numbers of the genomic location in the DNA sequence data depends on the overall viability (replication rate) of the cells as well as the proximity of the genomic location to the origin of replication (ori) as depicted in the FIG. 4. Here, the population refers to a set of replicating microbial cells either bacteria/ archaea (or any other organisms having a circular genome/chromosome and following a bi-directional replication process), whereas copy numbers refer to the absolute number of genomic loci present at any specific time point of replication. For example, considering replication process for one genome/ chromosome, a particular gene/ genomic loci located close to origin of replication (ori), where DNA replication has already taken place has two copies, whereas a gene/genomic loci located close to the terminus of replication (ter), where replication is yet to happen, has only one copy. However, the above-mentioned copy numbers are not to be confused with native "copy numbers" of a gene in a chromosome, which is akin to repeat regions or duplicate genes present in the chromosome.

FIG. 5 illustrates a representative linear equation which can be fitted with a read coverage data for two distinct genomic loci, the first phylogenetic marker gene (say *A*) and the second phylogenetic marker gene (say *B*), obtained through amplicon sequencing, and their location on the genome with respect to the origin of replication (ori) and the terminus of replication (ter), in accordance with some embodiments of the present disclosure. The present disclosure exploits the relationship between the chromosomal/ genomic location of the gene/genomic loci sequenced, and the number of DNA sequenced reads/fragments of the gene/genomic loci (also known as read coverage) detected from the DNA sequence data (DNA sequence fragment reads) to extrapolate the overall replication rate of the bacterial organism present in the microbiome sample. In general, an inversely proportional relationship between the read coverage of the genomic loci and its distance from the origin of replication (ori) can be anticipated. On the other hand, the replication rate is expected to determine the slope (*m*) of this linear function, when the circular genome is depicted in the linear scale placing the 'ori' at the origin of replication, i.e., setting the 'ori' at 'zero' of this linear scale as depicted in Fig. 5. It will be understood, if the cell(s) are not replicating, one would not expect to have a higher number of copies/coverage for the genomic-loci closer to the origin of replication (ori), hence a 'zero' slope can be expected. On the other hand, a quicker replication rate entails a steeper slope.

Referring to FIG.5, x- axis represents a distance from the origin of replication (ori) and y-axis represents a number of mapped genes or read coverage. A plurality of known parameters which includes $x_{ori}$ represents the location of the origin of replication (ori), $x_{ter}$ represents the location of the terminus of replication (ter), $x_A$ represents the distance of the first phylogenetic marker gene (say *A*) from the origin of replication (ori) or in other words its location on the genome, $x_B$ represents the distance of the second phylogenetic marker gene (say *B*) from the origin of replication (ori) or in other words its location on the genome, $y_A$ represents the read coverage data of the first phylogenetic marker gene (say *A*) and the $y_B$ represents the read coverage data of the second phylogenetic marker gene (say *B*), as interpreted from the sequencing experiment. Further, in one implementation a plurality of parameters to derive includes $y_{ori}$ which represents the estimated read coverage data at the origin of replication (ori), $y_{ter}$ which represents the estimated read coverage data at the terminus of replication (ter), and the slope *(m)* represented by $\frac{y_B - y_A}{x_B - x_A}$ obtained from the fitted linear function for any given bacterial organism comprised in the collected microbiome sample.

In an embodiment of the present disclosure, an estimation of the slope, or some other metric derived from the slope, e.g., the ratio between $y_{ori}$ and $y_{ter}$ can serve as a proxy of the replication rate wherein to estimate the slope of the envisaged linear function, the following information is minimally required -

i. Sequencing coverage of at least two genomic loci. (e.g., $y_A$ and $y_B$)

ii. Knowledge of the location of the two genomic loci in terms of distance from the origin of replication (ori) (e.g., $x_A$ and $x_B$).

iii. While the above two information would suffice for calculation of the envisaged slope when a single genome is under consideration, in a microbiome/ metagenomic context, it is imperative that both the genomic loci/ genes selected (*A* and *B*) are phylogenetic/taxonomic markers, so that they can be mapped back to their source genomes (species) with relative ease.

The present disclosure, in one implementation, relies on the bacterial CPN60 gene as a second genomic loci (in addition to the 16S rRNA gene as the first genomic loci), considering CPN60 is a house-keeping gene and is present in majority of bacterial species.

In the present disclosure, the selected genes for amplicon sequencing, viz. 16S and CPN60 are not always expected to be present as a single copy on the bacterial genome. In fact, there are many instances of multiple copies of 16S rRNA genes scattered around the bacterial genome. Accordingly, the total coverage of the gene found in multiple copies, for example, 16S rRNA (or CPN60 if required) needs to be normalized considering the genomic locations of the multiple copies of the gene when fitting the linear function. Further, certain bacterial species may have multiple genomes, multiple origins of replication, and in some cases asymmetric progression of replication forks. Hence, the envisaged linear function may need to be adjusted accordingly in such scenarios for these bacterial species, or even be updated to a higher order mathematical function suitable for describing the biological events. In the present disclosure, 16S rRNA and CPN60 has been chosen based on their property as a phylogenetic marker of bacterial taxonomies. However, the present disclosure can be implemented using any other genomic loci, provided they are ubiquitously present across all bacterial species (viz. 5S rRNA, rpoB, gyrB, rpoB, tufA etc.) or at least among the bacterial species in the environmental niche being studied/ of interest and can be reliably mapped back to the source species. Further, the present disclosure has been explained with the help of the taxonomic level 'bacterial species' wherein the knowledgebase can be curated to depict information at different taxonomic levels (even subspecies or operational taxonomic units i.e., OTUs), and so can the taxonomic classification/ binning process. Effectively in alternate implementations the present disclosure can be adapted to other relevant taxonomic levels as well.

A few of the potential applications of the present disclosure is listed below:

(a) Healthcare: In elucidation of the efficacy of (oral) probiotic (and prebiotic) formulations. While 16S sequencing might help in quantifying the total amount of probiotic bacterial load in the (stool) sample, it would not be able to detect the viability of the probiotic microbe, which is essential for its functional efficacy. The present disclosure, wherein the replication rate of the microbes can be concomitantly quantified with the taxonomic abundance is better suited for the purpose. The viability of the probiotic cells in storage (prior to use), for example to better estimate shelf-life, can also be evaluated using this method.

(b) Agriculture: Antimicrobials are often used to eliminate harmful bacteria and other pathogens from soil and/or plant parts. In a scenario, such as the optimization of dosage of such an antimicrobial, an estimation of the reduction in the (pathogenic) microbial population by the antimicrobial would be essential. While amplicon (16S) sequencing will not be able to distinguish between live and killed/attenuated microbes, the present disclosure which uses a combination of sequencing a taxonomic phylogenetic marker gene and a house-keeping gene for estimation of replication rate would be more appropriate.

(c) Industry: In addition to production of economically important metabolites, bioreactors are also used to study several biological phenomena, such as the formation of single/multi-species biofilms and or the effect of interventions on them. For example, the effect of a new drug on the release of microbes (from the biofilm) may be studied. The present disclosure would be helpful in quantifying both the taxonomic diversity of the microbes getting released from the biofilm as well as provide information on the changes in their growth rates.

(d) Environment: In a similar manner, the present disclosure can be employed to quantify the impact of stress (such as heavy rain, high temperature, acid leakage, oil spill etc.) or interventions (such as toxicities caused by use of oil spill dispersants, pesticides, fire-fighting foams, etc.) on the microbial population dynamics. As mentioned previously, in addition to the changes in taxonomic abundance, the present disclosure would also enable for tracking the growth rate of each of the bacterial groups comprising the microbial population.

(e) Effectively, this protocol can be used to reduce the number of sampling points in any longitudinal microbiome study, by virtue of being able to measure the dynamics of bacterial growth/ replication rate from a single sample.

The present disclosure also projects the slope of the fitted linear function as a proxy of the replication rate. In another implementation, other derived representations, such as 'peak-to-trough' ratio as used in one of the prior-arts, may be used to represent bacterial replication. Such representations would be of use when the assumed linearity of relationship between read coverage at a genomic loci and distance from origin of replication (ori) does not hold true in special cases mentioned above. The present disclosure has been projected for ascertaining bacterial taxonomic affiliations and replication rates, wherein the same can be extended for archaeal and eukaryotic organism. Similar to bacteria, archaea too harbour circular chromosomes and the present disclosure can be easily adapted for studying this domain of life by choosing appropriate phylogenetic marker/ ubiquitously present genes. In fact, choosing phylogenetic marker genes which are shared among different domains of life, can help in constructing a single experimental design to study both bacteria and archaeal distribution in an ecosystem. Eukaryotes, on the other hand, harbour linear chromosomes. However, each of the viable eukaryotic cells would contain mitochondria (also plastids for plant cells) with circular chromosomes, which are also expected to divide and replicate in-pace with the eukaryotic host cell. The presented disclosure can be adapted to track phylogenetic marker genes on these circular chromosomes to assess taxonomy and replication rate of a heterogeneous eukaryotic cell population. Further, the linear function can also be fitted in cases, where there are multiple copies of a same gene on the chromosome. For example, copy1, copy2, copy3 and so on, of the same genes can be considered as phylogenetic marker gene1, phylogenetic marker gene2, phylogenetic marker gene3 and so on, wherein in this case only one primer for PCR is required.

The present disclosure can be extended to alternate implementations which involves-

(1) other phylogenetic marker genes which includes 5S rRNA, gyrB, rpoB, tufA and on the like.
(2) organisms other than bacteria which includes archaeal and eukaryotic.
(3) other metrics derivable from the slope to represent the growth/ replication rates of the bacteria present
(4) different normalized values derived from the read coverage data (of first phylogenetic marker gene and second phylogenetic marker gene) to indicate the taxonomic abundance of the bacteria present.

The validation of the method of the present disclosure i.e., simultaneous interpretation of taxonomic distribution and replication rates (SITAR) of microbes constituting microbial communities is described in below sections.

The method of simultaneous interpretation of taxonomic distribution and replication rates (SITAR) of microbes constituting microbial communities was validated using a simulated amplicon sequencing data generated from previously sequenced deep WGS data (whole genome shotgun sequencing metagenomic data). The WGS data used for this purpose (hereafter referred to as "WGS-data") was obtained from a public sequence repository with the accession number PRJNA273761 (https://www.ncbi.nlm.nih.gov/bioproject/PRJNA273761/).

The targeted amplicon regions for generating the simulated amplicon sequencing data corresponded to the phylogenetic marker genes V4 region of 16S rRNA and Universal Target (UT) region of CPN60 for the current validation process. As would be understood, in WGS (Whole genome sequencing) experiments the sequenced DNA fragment reads are obtained from all regions of the bacterial genomes constituting the microbiome. Hence, sequenced reads corresponding to the selected phylogenetic marker genes (viz. 16S rRNA and CPN60) for the bacteria (present in the microbiome samples subjected to WGS experiments) are expected to be present in the dataset along with sequenced reads from other genomic regions. As would also be understood, WGS (Whole genome sequencing) data also allows derivation of bacterial replication rates and bacterial relative abundance in the microbiome sample as described in prior arts (Korem et al, Brown et al.), and these values (replication rates in terms of the metric bPTR) were available for the downloaded WGS dataset from prior journal article (Brown et al). The bacterial replication rates computed using the present method were compared with the previously reported bPTR values for validating the utility of the present method, which among others include obtaining the simultaneous estimate of bacterial diversity and replication rates at a significantly lower cost than WGS (Whole genome sequencing) sequencing and longitudinal amplicon sequencing experiments. WGS (Whole genome sequencing) derived bacterial relative abundances were also compared with relative abundances derived through the present method during validation.

The steps for calculation of the metrics enabling simultaneous interpretation of taxonomic distribution and replication rates (SITAR) of microbes constituting microbial communities, depicting the bacterial replication rates, and their relative abundance are described as follows. While the WGS (Whole genome sequencing) data had sequenced reads from multiple organisms across many sample datasets, the pre-computed bPTR values (using state of the art methods) were only available for 89 instances corresponding to four bacterial genomes (having both the phylogenetic marker genes selected for this validation), present in 51 microbiome samples. It is to be understood that in case of 'PTR' values such as kPTR (Korem et al) or bPTR (Brown et al), a value = 1 would indicate that the bacteria are in a non-replicative/not growing stage and a value < 1 is a theoretical impossibility and possibly an experimental artifact. Hence, data corresponding to 21 instances, wherein bPTR values were reported to be < 1, was not considered during the validation.

The core method of present disclosure involves fitting the read coverage of two phylogenetic marker genes (or regions thereof) into a straight line (as described earlier), and then subsequently deriving an estimate of the bacterial growth rate using the slope of the straight line. It will be understood that in case the two phylogenetic marker genes are located very close on the genome, small errors in the sequencing or read mapping process can amplify into major errors in calculation of the slope. Therefore, in this validation experiment, further 26 instances of previously available bPTR values were not considered, since they corresponded to 2 bacterial genomes (Enterobacter cloacae strain, n=15; Klebsiella oxytoca strain, n=11) wherein the effective location of the chosen phylogenetic marker genes - 16S rRNA and CPN60 are within <=5% of the distance between origin of replication (ori) and terminus of replication (ter) locations. A choice of a different set of phylogenetic marker genes can overcome this limitation in an alternate implementation.

Given that one of the objectives of the present disclosure was to compute and compare the relative abundance of the bacteria constituting the microbiome, only instances from those samples were retained for final validation wherein computation of relative abundances was possible. Therefore, the final validation set comprised of 22 instances of previously available bPTR values from 11 samples, wherein the two organisms (Clostridium perfringens ATCC 13124, n=11; Enterococcus faecalis OG1RF, n=11) were present in each of the samples. Simulated amplicon data was created (hereafter called "sim-data") for these 11 samples with intent of comparing efficiency of the newly proposed method against previously reported bPTR values and WGS derived abundances of bacteria from these 11 samples.

The WGS-data from 11 samples (Run accession numbers SRR1779125, SRR1779126, SRR1779134, SRR1779135, SRR1779139, SRR1779141, SRR1779147, SRR1779148, SRR1779149, SRR1779150, SRR1779152 ) were used for mapping metagenomic reads to the reference genomic data (pre-existing) of two organisms (using bowtie2), and the metagenomic fragment reads which mapped to the locations corresponding to the selected phylogenetic marker genes (i.e. 16S rRNA and CPN60) were considered to constitute the sim-data (simulated amplicon sequencing data).

The following commands were used to run bowtie2 program(ref) based mapping of WGS data onto reference genomic data.

Building reference genome database for organism1 (org1):

*bowtie2-build Reference_genome_org1.fna Reference_genome_org1_db bowtie2-inspect --summary Reference_genome_org1_db*
wherein Reference_genome_org1.fna: reference organism genome fasta file; *Reference_genome_org1_db is database build for bowtie2 mapping.*

Mapping metagenome reads to the created reference genome database:

*bowtie2 --end-to-end -x Reference_genome_org1_db -1 metagenome_sample_1_fwd.fastq -2 metagenome_sample_1_rev.fastq -S metagenome_sample_1_genome_1.sam -p 40 --no-unal samtools view -f 2 -h -bS metagenome _sample _1_genome_1.sam >*
*metagenome_sample_1_genome_1.bam*
*samtools sort metagenome*_sample_1_genome_1.*bam -o* metagenome.sorted_sample_1_genome_1.bam
*samtools index* metagenome.sorted_sample_1_genome_1.bam

wherein, *metagenome_sample_1_fwd.fastq* and *metagenome_sample_1_rev.fastq* corresponds to WGS data form metagenome sample_1 in fastq format from a paired end sequencing experiment. The suffixes 'fwd' and 'rev' are indicative of the files containing forward and reverse reads respectively.

*metagenome_sample_1_genome_1.sam* is SAM format of alignment (mapping). *metagenome*_sample_1_genome_1.*bam* is binary form of SAM alignment(mapping). metagenome.sorted_sample_1_genome_1.bam is sorted form of 'metagenome_sample_1_genome_1.*bam'* based on genome location to which it has been mapped.

In a similar manner, reference genome database creation was done for both the organisms and subsequently mapping was done for all 11 samples against created reference database(s).

In an embodiment of the present disclosure, the database creation and mapping command were run with default parameters for bowtie2. However, depending on the type of data other parameters and even other read mapping software tools can be used. In one implementation, the reference genomic data used for mapping in above step can constitute of only the genomic sequence(s) corresponding to the selected phylogenetic marker genes, and not the entire bacterial genome. In another implementation all these reference sequences for selected phylogenetic marker genes can be prepopulated into a single reference database for mapping while properly indexing their respective occurrences in this single reference database file.

From the simulated data, read coverages (i.e., number of sequenced nucleotide bases corresponding to a targeted sequenced region normalized by length of the sequenced region) for the selected phylogenetic marker gene regions (i.e., 16S rRNA and CPN60) were computed for each of the bacteria in all the samples. The below command was used to calculate the number of bases mapped on the particular location of 16S rRNA and CPN60 respectively.

*samtools depth metagenome.sorted sample_1_genome_1.bam -r genome_1:V4_region_start_position- V4 region end position -> metagenome_sample_1.genome_1.V4_region.info*

*samtools depth metagenome.sorted sample_1_genome_1.bam -r genome_1:CPN60_region_start_position- CPN60 region end position -> metagenome _sample_1.genome_1. CPN60_region.info*

wherein, *metagenome.sorted_sample_1_genome_1.bam is sorted alignment* of *metagenome sample 1 w.r.t. genome_1 location.*

*metagenome_sample_1.genome_1. V4_region.info contains the number of bases mapped to each position in the V4 region of genome1 from metagenome sample 1.*

*metagenome_sample_1.genome_1.CPN60 region.info contains the number of bases mapped to each position in the CPN60 region of genome1 from metagenome sample 1.*

In a similar manner, the number of bases mapped for both the phylogenetic marker genes for each of genome for all 11 samples was calculated, subsequently read coverages for selected phylogenetic marker genes were computed as mentioned earlier.

In an embodiment of the present disclosure, the locations of the phylogenetic marker genes were represented/ transformed in a pre-computed linear scale of 0-100, with respect to the origin of replication (ori) and the terminus of replication (ter) of the respective bacterial genomes, depicting the chromosome/genome of each bacterium. Further, sequences of the targeted phylogenetic marker genes were also stored. It is to be noted that this is a onetime step which is carried out for all known fully sequenced bacterial genomes and this information can be reused for all experiments deploying the proposed method of SITAR. Further, the creation of precomputed reference sequence database for all known fully sequenced bacterial genomes can be performed as a onetime step which can be used later for mapping/aligning amplicon sequenced reads when using the proposed method of SITAR.

In an embodiment of the present disclosure, average locations and coverages of the selected region of markers genes 16S rRNA and CPN60 from the concerned bacteria were calculated, whenever multiple copies of same gene existed in any of these bacteria. These average values represented effective coverage and effective location of both the phylogenetic marker genes (16S rRNA and CPN60) which were used to fit the linear equation of the form y = $mx$ + c for each bacterium in a particular sample, wherein y represents read coverage of a genomic location and $x$ is represented in the precomputed scale of 0-100, where the location of origin of replication (ori) is 0 and location of terminus of replication (ter) is 100. For each bacterium in a particular sample, considering the effective coverage of the two selected phylogenetic marker genes as '$y_A$' and '$y_B$' and their effective locations as '$x_A$' and '$x_B$' respectively, the following steps were used to arrive at the equation of a straight line, the steepness of whose slope 'm' would be dependent on the replication rate of the concerned bacterium in a particular sample. Subsequently, in order to compare the bPTR, which essentially represents the ratio between a peak coverage and trough coverage, an analogous metric estimating bacterial replication rates through SITAR method was computed as a ratio between '$y_{ori}$' and '$y_{ter}$' i.e., the imputed/estimated effective coverage at location 0 and 100 of the pre-computed linear scale depicting the chromosome of each bacterium.

Fitting linear equation to calculate coverage at origin ($y_{ori}$) and terminus ($y_{ter}$)

Generic equation of a straight line

$$y - y_A = \frac{y_B - y_A}{x_B - x_A} \cdot (x - x_A) \ldots (1)$$

Can be re-written as,

$$y = \frac{y_B - y_A}{x_B - x_A} \cdot x + y_1 - \frac{y_B - y_A}{x_A - x_B} \cdot x_A \ldots (2)$$

The above equation is of the form y = $m.x$ + c wherein, $m = \frac{y_B - y_A}{x_B - x_A}$, and $c = y_1 - \frac{y_B - y_A}{x_B - x_A} \cdot x_A$

The estimated read coverage at origin of replication (ori) can then be calculated as $y_{ori} = m \times 0 + c$ (given $x_{ori} = 0$)

$$\text{i.e., } y_{ori} = c \ldots (3)$$

Similarly, the estimated read coverage at terminus of replication (ter) can be calculated as $y_{ter} = m \times 100 + c \ldots (4)$

Certain boundary conditions were introduced to ensure that the estimated $y_{orl}$ and $y_{ter}$ values remain in biologically feasible ranges. For this purpose, in an example embodiment of the present disclosure, a large distribution of ratios of read coverages at 16S rRNA phylogenetic marker gene with respect to read coverages at the terminus of replication locations for all organisms was generated from pre-existing WGS data and the top 95$^{th}$ percentile value of this ratio (T) was noted. In cases, wherein computed $y_{ter}$ value was $<= \dfrac{y_{16s}}{T}$ (wherein $y_{16s}$ is effective coverage of 16S rRNA phylogenetic marker gene) a modified $y_{ter}$ value ( $y'_{ter}$ ) was computed as $y'_{ter} = \dfrac{y_{16s}}{T}$. Subsequently, keeping *'m'* unchanged, a modified $y_{ori}$ value ( $y'_{ori}$ ) was computed as $y'_{ori} = y'_{ter} - m \times 100$. The empirically derived value of T in case of the validation study of the present disclosure is 74.27, where this value may be re-calculated/ updated in another implementation based on availability of additional large scale metagenomic WGS (Whole genome sequencing) data.

From the above computed values of coverage data at origin and terminus for each bacterium, SITAR method's metric estimating bacterial replication rates was computed as mentioned below.

If, $y_{ter} > \dfrac{y_{16s}}{T}$ then, the metric is calculated as

$$SITAR = \frac{y_{ori}}{y_{ter}} \ldots\ldots\ldots(5)$$

Else if $y_{ter} \leq \dfrac{y_{16s}}{T}$ then $y'_{ter}$ and $y'_{ori}$ can be calculated as

$$y'_{ter} = \frac{y_{16s}}{T} \text{ and } y'_{ori} = y'_{ter} - m \times 100 \ldots\ldots\ldots(6)$$

and subsequently SITAR method's metric estimating bacterial replication rate can be computed as

$$SITAR = \frac{y'_{ori}}{y'_{ter}} \ldots\ldots\ldots(7)$$

In addition to the above steps a further constraint was added to keep the value of SITAR method's metric estimating bacterial replication rates in biologically feasible ranges wherein the minimum value of SITAR method's metric estimating bacterial replication rate was set at 1.

In an alternate implementation wherein more than two phylogenetic marker genes are available/considered for amplicon sequencing, the straight line (of the form *y = m.x +* c) mentioned above can also be obtained or fitted utilizing the coverage values of more than two phylogenetic marker genes while using linear regression.

Pearson's correlation coefficient (*r*) was computed between the WGS (Whole genome sequencing) derived bPTR values and SITAR method's metric estimating bacterial replication rates values, in order to determine the efficiency of the present method in estimating the bacterial growth rates, using amplicon sequencing based approach. Table 1 represents the imputed bacterial growth rates represented in form of values of SITAR method's metric estimating bacterial replication rate and the corresponding bPTR values as available from WGS (Whole genome sequencing) data for each genome in all samples. The correlation for these 22 instances corresponding to 11 samples and 2 organisms was observed to be 0.77 (p-value 2.7e-05) indicating the high efficiency of the SITAR method's metric estimating bacterial replication rate proposed in the present method in estimating bacterial growth rates or replication rates. Given the differences in ways of computing, bPTR and SITAR method's metric values are not expected to be in the same range/ scale. However, the high correlation shows that the SITAR method's metric estimating bacterial replication rates has almost equivalent efficiency as the WGS (Whole genome sequencing) derived bPTR values while performing a relative comparison of growth rates between different organisms across different samples.

Table 1. Imputed bacterial growth rates represented in form of SITAR method's metric estimating bacterial replication rate values and the corresponding bPTR values as available from WGS (Whole genome sequencing) data for each genome in all samples.

| Sample | Organism | bPTR | SITAR method's metric estimating bacterial replication rate |
|---|---|---|---|
| SRR1779148 | Clostridium perfringens ATCC 13124 | 1.9442 | 807.7945 |
| SRR1779126 | Clostridium perfringens ATCC 13124 | 1.8710 | 903.5825 |
| SRR1779152 | Clostridium perfringens ATCC 13124 | 2.4817 | 847.6301 |
| SRR1779135 | Clostridium perfringens ATCC 13124 | 1.2467 | 830.1449 |
| SRR1779149 | Clostridium perfringens ATCC 13124 | 2.0214 | 891.0501 |
| SRR1779147 | Clostridium perfringens ATCC 13124 | 1.4401 | 860.2601 |
| SRR1779134 | Clostridium perfringens ATCC 13124 | 1.3731 | 180.9049 |
| SRR1779125 | Clostridium perfringens ATCC 13124 | 1.8724 | 816.4361 |
| SRR1779150 | Clostridium perfringens ATCC 13124 | 1.8828 | 760.2436 |
| SRR1779141 | Clostridium perfringens ATCC 13124 | 1.6662 | 858.5374 |
| SRR1779139 | Clostridium perfringens ATCC 13124 | 2.1125 | 618.6617 |
| SRR1779148 | Enterococcus faecalis OG1RF | 1.1746 | 1.1019 |
| SRR1779126 | Enterococcus faecalis OG1RF | 1.0959 | 411.8528 |
| SRR1779152 | Enterococcus faecalis OG1RF | 1.1615 | 290.1880 |
| SRR1779135 | Enterococcus faecalis OG1RF | 1.0402 | 0.2476 |
| SRR1779149 | Enterococcus faecalis OG1RF | 1.1700 | 274.7649 |
| SRR1779147 | Enterococcus faecalis OG1RF | 1.1120 | 129.3966 |
| SRR1779134 | Enterococcus faecalis OG1RF | 1.0974 | 109.0128 |
| SRR1779125 | Enterococcus faecalis OG1RF | 1.2686 | 368.7346 |
| SRR1779150 | Enterococcus faecalis OG1RF | 1.2246 | 268.4932 |
| SRR1779141 | Enterococcus faecalis OG1RF | 1.1055 | 378.2114 |
| SRR1779139 | Enterococcus faecalis OG1RF | 1.0160 | 213.0724 |

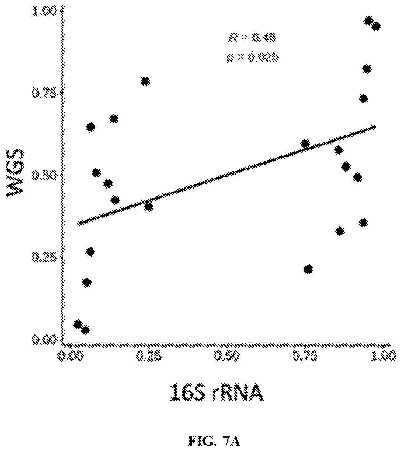

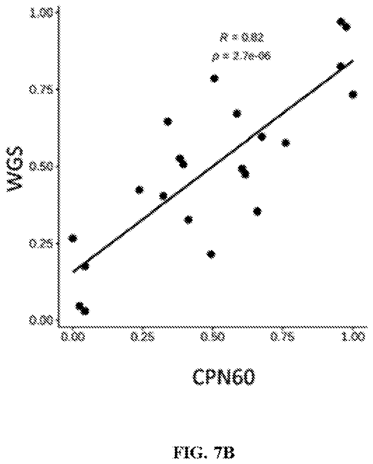

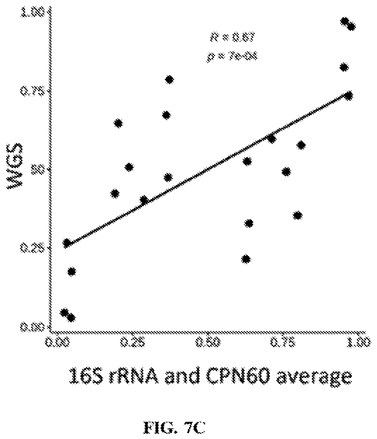

For comparing the accuracy of relative abundances obtained using the present method the relative abundance values were compared against WGS (Whole genome sequencing) derived relative abundances of the two organisms comprising the validation dataset. The WGS (Whole genome sequencing) based relative abundance for an organism in a given sample was computed as the ratio of coverage of the genome of that organism divided by the sum of the coverages of all the organism under consideration (in this case 2 organisms). The 16S rRNA and CPN60 based relative abundances for each of the organism were also computed in a similar manner. Table 2 represent the relative abundances of all the organism from WGS (Whole genome sequencing), 16S rRNA and CPN60 calculated in the current study for all the 11 samples. The relative abundance of the organism taking the average coverage values of 16S rRNA and CPN60 genes are also computed. The Pearson's correlation values were computed between WGS (Whole genome sequencing) derived relative abundances and (A) 16S rRNA, (B) CPN60, (C) average of 16S rRNA and CPN60 derived relative abundances, which are depicted in FIG.7A through 7C. The computed Pearson's correlation coefficient (r) value between WGS (Whole genome sequencing) derived relative abundance and 16S rRNA based relative abundance is 0.48 (p value = 0.025). Whereas the computed r value between WGS (Whole genome sequencing) derived relative abundance and CPN60 based relative abundance is 0.82 (p value = 2.7e-06). Additionally, the computed r values between WGS derived relative abundance and average of 16S rRNA and CPN60 based relative abundances is 0.67 (p value = 7e-04). Please note that, it is known in the art that many a times WGS (Whole genome sequencing) derived abundances may not be identical with state of art 16S rRNA amplicon sequencing based relative abundance. This could be due to variety of factors including read mapping artifacts arising from copy number variability of 16S rRNA genes.

In the present validation study, it is observed that 16S rRNA derived relative abundances and WGS (Whole genome sequencing) derived relative abundances have a decent or reasonable and statistically significant correlation.

However, the correlation observed between CPN60 and WGS (Whole genome sequencing) derived relative abundances was much strongly correlated (r = 0.82). Also average of relative abundances derived from 16S rRNA and CPN60 were better correlated to WGS (Whole genome sequencing) derived abundances than only 16S rRNA based abundance. This indicates considering deriving relative abundance of bacteria in a microbiome sample using information from amplicon sequencing of additional phylogenetic marker genes (in addition state of the art 16S rRNA amplicons) can help in improved estimates.

Table2. The table contains relative abundances of the two organisms derived from WGS (Whole genome sequencing), 16S rRNA, CPN60 and average of 16S rRNA and CPN60 read coverages, calculated in the current study for all the 11 samples.

| Sample | Organism | WGS relative abundance | 16s rRNA relative abundance | CPN60 relative abundance | Average of 16S rRNA and CPN60 relative abundances |
|---|---|---|---|---|---|
| SRR1779148 | Clostridium perfringens ATCC 13124 | 0.4929 | 0.9181 | 0.6046 | 0.7614 |
| SRR1779126 | Clostridium perfringens ATCC 13125 | 0.7338 | 0.9364 | 1.0000 | 0.9682 |
| SRR1779152 | Clostridium perfringens ATCC 13126 | 0.2145 | 0.7604 | 0.4944 | 0.6274 |
| SRR1779135 | Clostridium perfringens ATCC 13127 | 0.5256 | 0.8801 | 0.3834 | 0.6317 |
| SRR1779149 | Clostridium perfringens ATCC 13128 | 0.3542 | 0.9356 | 0.6601 | 0.7979 |
| SRR1779147 | Clostridium perfringens ATCC 13129 | 0.3281 | 0.8619 | 0.4135 | 0.6377 |
| SRR1779134 | Clostridium perfringens ATCC 13130 | 0.9702 | 0.9531 | 0.9569 | 0.9550 |
| SRR1779125 | Clostridium perfringens ATCC 13131 | 0.8248 | 0.9488 | 0.9566 | 0.9527 |
| SRR1779150 | Clostridium perfringens ATCC 13132 | 0.5766 | 0.8575 | 0.7614 | 0.8094 |
| SRR1779141 | Clostridium perfringens ATCC 13133 | 0.9536 | 0.9770 | 0.9763 | 0.9767 |
| SRR1779139 | Clostridium perfringens ATCC 13134 | 0.5965 | 0.7499 | 0.6763 | 0.7131 |
| SRR1779148 | Enterococcus faecalis OG1RF | 0.5071 | 0.0819 | 0.3954 | 0.2386 |
| SRR1779126 | Enterococcus faecalis OG1RF | 0.2662 | 0.0636 | 0.0000 | 0.0318 |
| SRR1779152 | Enterococcus faecalis OG1RF | 0.7855 | 0.2396 | 0.5056 | 0.3726 |

(continued)

| Sample | Organism | WGS relative abundance | 16s rRNA relative abundance | CPN60 relative abundance | Average of 16S rRNA and CPN60 relative abundances |
|---|---|---|---|---|---|
| SRR1779135 | Enterococcus faecalis OG1RF | 0.4744 | 0.1199 | 0.6166 | 0.3683 |
| SRR1779149 | Enterococcus faecalis OG1RF | 0.6458 | 0.0644 | 0.3399 | 0.2021 |
| SRR1779147 | Enterococcus faecalis OG1RF | 0.6719 | 0.1381 | 0.5865 | 0.3623 |
| SRR1779134 | Enterococcus faecalis OG1RF | 0.0298 | 0.0469 | 0.0431 | 0.0450 |
| SRR1779125 | Enterococcus faecalis OG1RF | 0.1752 | 0.0512 | 0.0434 | 0.0473 |
| SRR1779150 | Enterococcus faecalis OG1RF | 0.4234 | 0.1425 | 0.2386 | 0.1906 |
| SRR1779141 | Enterococcus faecalis OG1RF | 0.0464 | 0.0230 | 0.0237 | 0.0233 |
| SRR1779139 | Enterococcus faecalis OG1RF | 0.4035 | 0.2501 | 0.3237 | 0.2869 |

The written description describes the subject matter herein to enable any person skilled in the art to make and use the embodiments. The scope of the subject matter embodiments is defined by the claims and may include other modifications that occur to those skilled in the art. Such other modifications are intended to be within the scope of the claims if they have similar elements that do not differ from the literal language of the claims or if they include equivalent elements with insubstantial differences from the literal language of the claims.

It is to be understood that the scope of the protection is extended to such a program and in addition to a computer-readable means having a message therein; such computer-readable storage means contain program-code means for implementation of one or more steps of the method, when the program runs on a server or mobile device or any suitable programmable device. The hardware device can be any kind of device which can be programmed including e.g., any kind of computer like a server or a personal computer, or the like, or any combination thereof. The device may also include means which could be e.g., hardware means like e.g., an application-specific integrated circuit (ASIC), a field-programmable gate array (FPGA), or a combination of hardware and software means, e.g., an ASIC and an FPGA, or at least one microprocessor and at least one memory with software processing components located therein. Thus, the means can include both hardware means and software means. The method embodiments described herein could be implemented in hardware and software. The device may also include software means. Alternatively, the embodiments may be implemented on different hardware devices, e.g., using a plurality of CPUs.

The embodiments herein can comprise hardware and software elements. The embodiments that are implemented in software include but are not limited to, firmware, resident software, microcode, etc. The functions performed by various components described herein may be implemented in other components or combinations of other components. For the purposes of this description, a computer-usable or computer readable medium can be any apparatus that can comprise, store, communicate, propagate, or transport the program for use by or in connection with the instruction execution system, apparatus, or device.

The illustrated steps are set out to explain the exemplary embodiments shown, and it should be anticipated that ongoing technological development will change the manner in which particular functions are performed. These examples are presented herein for purposes of illustration, and not limitation. Further, the boundaries of the functional building blocks have been arbitrarily defined herein for the convenience of the description. Alternative boundaries can be defined so long as the specified functions and relationships thereof are appropriately performed. Alternatives (including equivalents, extensions, variations, deviations, etc., of those described herein) will be apparent to persons skilled in the relevant art(s) based on the teachings contained herein. Such alternatives fall within the scope of the disclosed embodiments. Also, the words "comprising," "having," "containing," and "including," and other similar forms are intended to be equivalent in meaning and be open ended in that an item or items following any one of these words is not meant to be an exhaustive listing of such item or items, or meant to be limited to only the listed item or items. It must also be noted that as used herein and in the appended claims, the singular forms "a," "an," and "the" include plural references unless the context clearly dictates otherwise.

Furthermore, one or more computer-readable storage media may be utilized in implementing embodiments consistent with the present disclosure. A computer-readable storage medium refers to any type of physical memory on which information or data readable by a processor may be stored. Thus, a computer-readable storage medium may store instructions for execution by one or more processors, including instructions for causing the processor(s) to perform steps or stages consistent with the embodiments described herein. The term "computer-readable medium" should be understood to include tangible items and exclude carrier waves and transient signals, i.e., be non-transitory. Examples include random access memory (RAM), read-only memory (ROM), volatile memory, nonvolatile memory, hard drives, CD ROMs, DVDs, flash drives, disks, and any other known physical storage media.

It is intended that the disclosure and examples be considered as exemplary only, with a true scope of disclosed embodiments being indicated by the following claims.

**Claims**

1. A method (200) for simultaneous interpretation of taxonomic distribution and replication rates of microbes constituting microbial communities, the method comprising:

   collecting a microbiome sample from a given environment (202);
   extracting bacterial genomic DNA (Deoxyribonucleic Acid) from a plurality of bacterial organisms constituting the collected microbiome sample (204);
   performing an amplicon sequencing by a PCR (Polymerase chain reaction) amplification module (106) and a sequencer (108), on the extracted bacterial genomic DNA comprising at least one of (i) targeting two or more phylogenetic marker genes and (ii) selecting a portion from each of the two or more phylogenetic marker genes to obtain a plurality of DNA sequence fragment reads, wherein the two or more phylogenetic marker genes are found in genomes of organisms, and which are used for identification of the taxonomic lineage of the organism (206);
   mapping, by a processor (114), the plurality of the DNA sequence fragment reads to a precomputed reference sequence database of a plurality of available completely sequenced bacterial genomes (208);
   identifying, by the processor (114), a plurality of bacterial organisms in the collected microbiome sample and assigning a taxonomic classification to the identified plurality of bacterial organisms based on the mapping of the plurality of the DNA sequence fragment reads to the precomputed reference sequence database (210);
   measuring, by the processor (114), read coverage at the genomic locations of the two or more phylogenetic marker genes for the plurality of identified bacterial organisms based on the mapping of the plurality of the DNA sequence fragment reads to the precomputed reference sequence database, and wherein the measured read coverage is used for the interpretation of the taxonomic distribution of the plurality of bacterial organisms identified from the collected microbiome sample (212);
   fitting, by the processor (114), a linear function of the form $y = mx + c$ for each of the plurality of bacterial organisms by using the measured read coverage and the information of the genomic locations corresponding to the two or more phylogenetic marker genes with respect to an origin of replication (ori) and a terminus of replication (ter) specific to each of the plurality of bacterial organisms identified from the collected microbiome sample (214);
   obtaining, by the processor (114), slope ($m$) from the fitted linear function of the form $y = mx + c$ for each of the plurality of bacterial organisms identified from the collected microbiome sample (216);
   estimating, by the processor (114), for each of the plurality of bacterial organisms identified from the collected microbiome sample an expected read coverage at the origin of replication $y_{ori}$ using the slope ($m$) obtained and the value c from the fitted linear function (218);
   estimating, by the processor (114), for each of the plurality of bacterial organisms identified from the collected microbiome sample an expected read coverage at the terminus of replication $y_{ter}$ using the slope ($m$) obtained and the value c from the fitted linear function (220); and
   interpreting, by a processor (114), for each of the plurality of bacterial organisms identified from the collected microbiome sample a replication rate using at least one of (i) slopes ($m$) and (ii) ratio of $\dfrac{y_{ori}}{y_{ter}}$ (222).

2. The processor implemented method of claim 1, wherein the given environment for collecting the microbiome sample comprises:

   (i) collecting the microbiome sample from one of the body sites of the human including gut, skin, hair, nasopharynx and from body fluids including saliva, urine, blood, stool, sputum, and cerumen;

(ii) collecting the microbiome sample from one of the body sites of the animal including gut, skin, hair, nasopharynx and from body fluids including saliva, urine, blood, stool, sputum, and cerumen;

(iii) collecting the microbiome sample from different parts of a plant, viz., endosphere, rhizosphere, rhizoplane, leaf, fruit, seed and from plant and plant product extracts;

(iv) collecting the microbiome sample from environmental sources including sewage, bioreactor, river bed, ocean bed and air; and

(v) collecting the microbiome sample from stored biological or organic material, including raw food, processed food, food grains, natural product derived drugs and probiotic formulations intended for therapeutic use.

3. The processor implemented method of claim 1, wherein the two or more phylogenetic marker genes comprises 16S rRNA, CPN60, 5S rRNA, gyrB, rpoB, and tufA, and wherein the precomputed reference sequence database comprises of the distance of the phylogenetic marker genes from the origin of replication (ori) and the terminus of replication (ter) of a circular chromosome from all available completely sequenced bacterial genomes.

4. The processor implemented method of claim 3, wherein the precomputed reference sequence database created further comprises:

(i) ascertaining the genomic location of the phylogenetic marker genes for all available completely sequenced bacterial genomes;

(ii) acquiring historical genomic location of the origin of replication (ori) and the terminus of replication (ter) for all available completely sequenced bacterial genomes;

(iii) creating the genomic location database of the phylogenetic marker genes in terms of the distance from the origin of replication (ori) and the terminus of replication (ter); and

(iv) the genomic locations of the phylogenetic marker genes were represented in a pre-computed linear scale of 0-100 with respect to the locations of the origin of replication (ori) and the terminus of replication (ter) of the respective bacterial genomes constituting the precomputed reference sequence database,

and wherein the step of creating the precomputed reference sequence database further comprises creating a genomic sequence database which further comprises sequences of the targeted phylogenetic marker genes from all the available completely sequenced bacterial genomes.

5. The processor implemented method of claim 1, wherein the taxonomic distribution of the plurality of bacterial organisms identified from the collected microbiome sample is interpreted as the relative abundance of at least one of (i) measured read coverage of one of the phylogenetic marker genes, and (ii) an average of the measured read coverage of the two or more phylogenetic marker genes.

6. The processor implemented method of claim 1, wherein the step of fitting a linear function further comprises at least one of:

$$m = \frac{y_B - y_A}{x_B - x_A} \quad \text{and} \quad c = y_A - \frac{y_B - y_A}{x_B - x_A} \times x_A$$

fitting the linear function of the form $y = mx + c$ wherein , wherein $y_A$ and $y_B$ represents the measured read coverage for two phylogenetic marker genes $A$ and $B$ respectively, and $x_A$ and $x_B$ represents the corresponding genomic locations of the two phylogenetic marker genes $A$ and $B$ respectively, and

fitting the linear function of the form $y = mx + c$ using a linear regression wherein measured read coverages $(y_A, y_B, y_C... y_N)$ for more than two phylogenetic marker genes $(A, B, C ...... N)$ and corresponding genomic locations $(x_A, x_B, x_C... x_N)$ for more than two phylogenetic marker genes $(A, B, C ...... N)$ are considered.

7. The processor implemented method of claim 1, wherein the targeted phylogenetic marker genes are selected such that the effective locations of the marker genes are separated by a distance >=5% of the distance between origin of replication (ori) and terminus of replication (ter) locations in a majority of the bacterial organisms that are expected to be present in an environment from which the microbiome sample has been collected, wherein calculating an effective location of a phylogenetic marker gene on a bacterial genome comprises calculation of an average of the locations for the one or more copies of the same phylogenetic marker gene, and calculating an effective read coverage of a phylogenetic marker gene on a bacterial genome comprises calculation of an average read coverage for the one or more copies of the same phylogenetic marker gene, when multiple copies of same phylogenetic marker genes are present in any of the plurality of bacterial organisms identified from the collected microbiome sample, and wherein effective read coverage and effective location of the two or more phylogenetic marker genes are used to fit the linear equation of the form y = *mx + c*.

8. The processor implemented method of claim 1, wherein estimating the expected read coverage at the origin of replication $y_{ori}$ and an expected read coverage at the terminus of replication $y_{ter}$ further comprises generating a large distribution of ratios of read coverage at 16S rRNA with respect to read coverage at the terminus of replication (ter) for a plurality of bacteria from pre-existing whole genome shotgun (WGS) sequenced data and noting the top 95th percentile value of the ratio (*T*), wherein this empirically derived value of *T* is used to ensure that estimated $y_{ori}$ and $y_{ter}$ are within biologically feasible ranges in the following manner,

if the estimated $y_{ter}$ value is $<= \dfrac{y_{16s}}{T}$ a modified value ($y'_{ter}$) is computed as $y'_{ter} = \dfrac{y_{16s}}{T}$, and a modified $y_{ori}$ value ($y'_{ori}$) is computed subsequently as $y'_{ori} = y'_{ter} - m \times 100$ and wherein $y_{16s}$ represents the effective coverage of 16S rRNA marker gene, and wherein the ratio T can be calculated as a ratio of read coverage at any other selected genomic region with respect to read coverage at the terminus of replication for the plurality of bacteria from pre-existing whole genome shotgun (WGS) sequenced data.

9. A system (100) for simultaneous interpretation of taxonomic distribution and replication rates of microbes constituting microbial communities, the system comprises:

   a sample collection module (102) for collecting the microbiome sample from a given environment;
   a DNA extraction module (104) for extracting bacterial genomic DNA from a plurality of bacterial organisms constituting the collected microbiome sample;
   a PCR amplification module (106) and a sequencer (108) for performing an amplicon sequencing, on the extracted bacterial genomic DNA comprising at least one of (i) targeting two or more phylogenetic marker genes and (ii) selecting a portion from each of the two or more phylogenetic marker genes to obtain a plurality of DNA sequence fragment reads, wherein the phylogenetic marker genes are found in genomes of organisms, and which are used for identification of the taxonomic lineage of the organism;
   a memory (116);
   and a processor (114) in communication with the memory (116), wherein the processor configured to perform the steps of:

      mapping the plurality of the DNA sequence fragment reads to a precomputed reference sequence database of a plurality of available completely sequenced bacterial genomes;
      identifying a plurality of bacterial organisms in the collected microbiome sample and assigning a taxonomic classification to the identified plurality of bacterial organisms based on the mapping of the plurality of the DNA sequence fragment reads to the precomputed reference sequence database;
      measuring read coverage at the genomic locations of the two or more phylogenetic marker genes for the plurality of identified bacterial organisms based on the mapping of the plurality of the DNA sequence fragment reads to the precomputed reference sequence database, and wherein the measured read coverage is used for the interpretation of the taxonomic distribution of the plurality of bacterial organisms identified from the collected microbiome sample;
      fitting a linear function of the form y = *mx + c* for each of the plurality of bacterial organisms by using the measured read coverage and the information of the genomic locations corresponding to the two or more phylogenetic marker genes with respect to an origin of replication (ori) and a terminus of replication (ter) specific to each of the plurality of bacterial organisms identified from the collected microbiome sample;
      obtaining a slope *(m)* from the fitted linear function of the form *y = mx + c* for each of the plurality of bacterial organisms identified from the collected microbiome sample;
      estimating for each of the plurality of bacterial organisms identified from the collected microbiome sample an expected read coverage at the origin of replication $y_{ori}$ using the slope *(m)* obtained and the value c from the fitted linear function;
      estimating for each of the plurality of bacterial organisms identified from the collected microbiome sample an expected read coverage at the terminus of replication $y_{ter}$ using the slope *(m)* obtained and the value c from the fitted linear function; and
      interpreting for each of the plurality of bacterial organisms identified from the collected microbiome sample a replication rate using at least one of (i) slopes *(m)* and (ii) ratio of $\dfrac{y_{ori}}{y_{ter}}$.

10. The system of claim 9, wherein the two or more phylogenetic marker genes comprises 16S rRNA, CPN60, 5S rRNA, gyrB, rpoB, and tufA, and wherein the precomputed reference sequence database comprises of the distance of the phylogenetic marker genes from the origin of replication (ori) and the terminus of replication (ter) of a circular chromosome from all available completely sequenced bacterial genomes.

11. The system of claim 10, wherein the precomputed reference sequence database created further comprises:

    (i) ascertaining the genomic location of the phylogenetic marker genes for all available completely sequenced bacterial genomes;

    (ii) acquiring historical genomic location of the origin of replication (ori) and the terminus of replication (ter) for all available completely sequenced bacterial genomes;

    (iii) creating the genomic location database of the phylogenetic marker genes in terms of the distance from the origin of replication (ori) and the terminus of replication (ter); and

    (iv) the genomic locations of the phylogenetic marker genes were represented in a pre-computed linear scale of 0-100 with respect to the locations of the origin of replication (ori) and the terminus of replication (ter) of the respective bacterial genomes constituting the precomputed reference sequence database,

and wherein the step of creating the precomputed reference sequence database further comprises creating a genomic sequence database which further comprises sequences of the targeted phylogenetic marker genes from all the available completely sequenced bacterial genomes.

12. The system of claim 9, wherein the step of fitting a linear function further comprises at least one of:

$$m = \frac{y_B - y_A}{x_B - x_A} \qquad c = y_A - \frac{y_B - y_A}{x_B - x_A} \times x_A$$

fitting the linear function of the form $y = mx + c$ wherein and , wherein $y_A$ and $y_B$ represents the measured read coverage for two phylogenetic marker genes $A$ and $B$ respectively, and $x_A$ and $x_B$ represents the corresponding genomic locations of the two phylogenetic marker genes $A$ and $B$ respectively; and

fitting the linear function of the form $y = mx + c$ using a linear regression wherein measured read coverages $(y_A, y_B, y_C... y_N)$ for more than two phylogenetic marker genes $(A, B, C ...... N)$ and corresponding genomic locations $(x_A, x_B, x_C... x_N)$ for more than two phylogenetic marker genes $(A, B, C ...... N)$ are considered.

13. The system of claim 9, wherein the targeted phylogenetic marker genes are selected such that the effective locations of the marker genes are separated by a distance >=5% of the distance between origin of replication (ori) and terminus of replication (ter) locations in a majority of the bacterial organisms that are expected to be present in an environment from which the microbiome sample has been collected, wherein calculating an effective location of a phylogenetic marker gene on a bacterial genome comprises calculation of an average of the locations for the one or more copies of the same phylogenetic marker gene, and calculating an effective read coverage of a phylogenetic marker gene on a bacterial genome comprises calculation of an average read coverage for the one or more copies of the same phylogenetic marker gene, when multiple copies of same phylogenetic marker genes are present in any of the plurality of bacterial organisms identified from the collected microbiome sample, and wherein effective read coverage and effective location of the two or more phylogenetic marker genes are used to fit the linear equation of the form $y = mx + c$.

14. One or more non-transitory machine-readable information storage mediums comprising one or more instructions which when executed by one or more hardware processors cause:

    collecting a microbiome sample from a given environment (202);

    extracting bacterial genomic DNA (Deoxyribonucleic Acid) from a plurality of bacterial organisms constituting the collected microbiome sample (204);

    performing an amplicon sequencing by a PCR (Polymerase chain reaction) amplification module (106) and a sequencer (108), on the extracted bacterial genomic DNA comprising at least one of (i) targeting two or more phylogenetic marker genes and (ii) selecting a portion from each of the two or more phylogenetic marker genes to obtain a plurality of DNA sequence fragment reads, wherein the two or more phylogenetic marker genes are found in genomes of organisms, and which are used for identification of the taxonomic lineage of the organism (206);

    mapping the plurality of the DNA sequence fragment reads to a precomputed reference sequence database of a plurality of available completely sequenced bacterial genomes (208);

    identifying a plurality of bacterial organisms in the collected microbiome sample and assigning a taxonomic classification to the identified plurality of bacterial organisms based on the mapping of the plurality of the DNA sequence fragment reads to the precomputed reference sequence database (210);

measuring read coverage at the genomic locations of the two or more phylogenetic marker genes for the plurality of identified bacterial organisms based on the mapping of the plurality of the DNA sequence fragment reads to the precomputed reference sequence database, and wherein the measured read coverage is used for the interpretation of the taxonomic distribution of the plurality of bacterial organisms identified from the collected microbiome sample (212);

fitting a linear function of the form y = mx + c for each of the plurality of bacterial organisms by using the measured read coverage and the information of the genomic locations corresponding to the two or more phylogenetic marker genes with respect to an origin of replication (ori) and a terminus of replication (ter) specific to each of the plurality of bacterial organisms identified from the collected microbiome sample (214);

obtaining slope *(m)* from the fitted linear function of the form *y = mx + c* for each of the plurality of bacterial organisms identified from the collected microbiome sample (216);

estimating for each of the plurality of bacterial organisms identified from the collected microbiome sample an expected read coverage at the origin of replication $y_{ori}$ using the slope (*m*) obtained and the value *c* from the fitted linear function (218);

estimating for each of the plurality of bacterial organisms identified from the collected microbiome sample an expected read coverage at the terminus of replication $y_{ter}$ using the slope *(m)* obtained and the value c from the fitted linear function (220); and

interpreting for each of the plurality of bacterial organisms identified from the collected microbiome sample a replication rate using at least one of (i) slopes *(m)* and (ii) ratio of $\dfrac{y_{ori}}{y_{ter}}$ (222).

15. The one or more non-transitory machine-readable information storage mediums of claim 14, wherein the step of fitting a linear function comprises at least one of:

fitting the linear function of the form y = mx + c wherein $m = \dfrac{y_B - y_A}{x_B - x_A}$ and $c = y_A - \dfrac{y_B - y_A}{x_B - x_A} \times x_A$ , wherein $y_A$ and $y_B$ represents the measured read coverage for two phylogenetic marker genes A and B respectively, and $x_A$ and $x_B$ represents the corresponding genomic locations of the two phylogenetic marker genes A and B respectively, and

fitting the linear function of the form y = mx + c using a linear regression wherein measured read coverages ($y_A$, $y_B$, $y_C$... $y_N$) for more than two phylogenetic marker genes *(A, B, C ...... N)* and corresponding genomic locations *($x_A$, $x_B$, $x_C$... $x_N$)* for more than two phylogenetic marker genes *(A, B, C ...... N)* are considered.


**Patentansprüche**

1. Verfahren (200) zur gleichzeitigen Interpretation der taxonomischen Verteilung und der Replikationsraten von Mikroben, die mikrobielle Gemeinschaften bilden, wobei das Verfahren umfasst:

Sammeln einer Mikrobiomprobe aus einer gegebenen Umgebung (202);

Extrahieren bakterieller genomischer DNA (Desoxyribonukleinsäure) aus einer Mehrzahl bakterieller Organismen, die die gesammelte Mikrobiomprobe (204) bilden;

Durchführen einer Amplikonsequenzierung durch ein PCR (Polymerasekettenreaktion)-Amplifikationsmodul (106) und einen Sequenzer (108) an der extrahierten bakteriellen genomischen DNA, umfassend mindestens eines von (i) Abzielen auf zwei oder mehr phylogenetische Markergene und (ii) Auswählen eines Teils aus jedem der zwei oder mehr phylogenetischen Markergene, um eine Mehrzahl von DNA-Sequenzfragment-Reads zu erhalten, wobei die zwei oder mehr phylogenetischen Markergene in Genomen von Organismen gefunden werden und die zur Identifikation der taxonomischen Abstammung des Organismus (206) verwendet werden;

Abbilden, durch einen Prozessor (114), der Mehrzahl der DNA-Sequenzfragment-Reads auf eine vorberechnete Referenzsequenzdatenbank einer Mehrzahl verfügbarer vollständig sequenzierter bakterieller Genome (208);

Identifizieren, durch den Prozessor (114), einer Mehrzahl bakterieller Organismen in der gesammelten Mikrobiomprobe und Zuweisen einer taxonomischen Klassifikation zu der identifizierten Mehrzahl bakterieller Organismen basierend auf dem Abbilden der Mehrzahl der DNA-Sequenzfragment-Reads auf die vorberechnete Referenzsequenzdatenbank (210);

Messen, durch den Prozessor (114), der Leseabdeckung an den genomischen Positionen der zwei oder mehr phylogenetischen Markergene für die Mehrzahl identifizierter bakterieller Organismen basierend auf dem Abbilden der Mehrzahl der DNA-Sequenzfragment-Reads auf die vorberechnete Referenzsequenzdatenbank, und wobei die gemessene Leseabdeckung für die Interpretation der taxonomischen Verteilung der Mehrzahl bakterieller Organismen verwendet wird, die aus der gesammelten Mikrobiomprobe (212) identifiziert wurden; Anpassen, durch den Prozessor (114), einer linearen Funktion der Form y = mx + c für jeden der Mehrzahl bakterieller Organismen unter Verwendung der gemessenen Leseabdeckung und der Informationen der genomischen Positionen, die den zwei oder mehr phylogenetischen Markergenen entsprechen, in Bezug auf einen Replikationsursprung (ori) und einen Replikationsterminus (ter), die für jeden der Mehrzahl bakterieller Organismen spezifisch sind, die aus der gesammelten Mikrobiomprobe (214) identifiziert wurden;

Erhalten, durch den Prozessor (114), einer Steigung ($m$) aus der angepassten linearen Funktion der Form $y = mx + c$ für jeden der Mehrzahl bakterieller Organismen, die aus der gesammelten Mikrobiomprobe (216) identifiziert wurden;

Schätzen, durch den Prozessor (114), für jeden der Mehrzahl bakterieller Organismen, die aus der gesammelten Mikrobiomprobe identifiziert wurden, einer erwarteten Leseabdeckung am Replikationsursprung $y_{ori}$ unter Verwendung der erhaltenen Steigung *(m)* und des Werts c aus der angepassten linearen Funktion (218);

Schätzen, durch den Prozessor (114), für jeden der Mehrzahl bakterieller Organismen, die aus der gesammelten Mikrobiomprobe identifiziert wurden, einer erwarteten Leseabdeckung am Replikationsterminus $y_{ter}$ unter Verwendung der erhaltenen Steigung *(m)* und des Werts c aus der angepassten linearen Funktion (220); und

Interpretieren, durch einen Prozessor (114), für jeden der Mehrzahl bakterieller Organismen, die aus der gesammelten Mikrobiomprobe identifiziert wurden, einer Replikationsrate unter Verwendung von mindestens einem von (i) Steigungen *(m)* und (ii) einem Verhältnis von $\dfrac{y_{ori}}{y_{ter}}$ (222).

2. Prozessorimplementiertes Verfahren nach Anspruch 1, wobei die gegebene Umgebung zum Sammeln der Mikrobiomprobe Folgendes umfasst:

(i) Sammeln der Mikrobiomprobe von einer der Körperstellen des Menschen, einschließlich Darm, Haut, Haar, Nasopharynx, und von Körperflüssigkeiten, einschließlich Speichel, Urin, Blut, Stuhl, Sputum und Cerumen;
(ii) Sammeln der Mikrobiomprobe von einer der Körperstellen des Tiers, einschließlich Darm, Haut, Haar, Nasopharynx, und von Körperflüssigkeiten, einschließlich Speichel, Urin, Blut, Stuhl, Sputum und Cerumen;
(iii) Sammeln der Mikrobiomprobe von verschiedenen Teilen einer Pflanze, nämlich Endosphäre, Rhizosphäre, Rhizoplane, Blatt, Frucht, Samen und aus Pflanzen- und Pflanzenproduktextrakten;
(iv) Sammeln der Mikrobiomprobe aus Umweltquellen, einschließlich Abwasser, Bioreaktor, Flussbett, Meeresboden und Luft; und
(v) Sammeln der Mikrobiomprobe aus gelagertem biologischem oder organischem Material, einschließlich Rohnahrung, verarbeiteter Nahrung, Nahrungsmittelkörnern, von Naturprodukten abgeleiteten Arzneimitteln und probiotischen Formulierungen, die zur therapeutischen Verwendung bestimmt sind.

3. Prozessorimplementiertes Verfahren nach Anspruch 1, wobei die zwei oder mehr phylogenetischen Markergene 16S rRNA, CPN60, 5S rRNA, gyrB, rpoB und tufA umfassen, und wobei die vorberechnete Referenzsequenzdatenbank den Abstand der phylogenetischen Markergene vom Replikationsursprung (ori) und dem Replikationsterminus (ter) eines kreisförmigen Chromosoms von allen verfügbaren vollständig sequenzierten bakteriellen Geomen umfasst.

4. Prozessorimplementiertes Verfahren nach Anspruch 3, wobei die erstellte vorberechnete Referenzsequenzdatenbank ferner Folgendes umfasst:

(i) Ermitteln der genomischen Position der phylogenetischen Markergene für alle verfügbaren vollständig sequenzierten bakteriellen Genome;
(ii) Erfassen einer historischen genomischen Position des Replikationsursprungs (ori) und des Replikationsterminus (ter) für alle verfügbaren vollständig sequenzierten bakteriellen Genome;
(iii) Erstellen der genomischen Positionsdatenbank der phylogenetischen Markergene in Bezug auf den Abstand vom Replikationsursprung (ori) und dem Replikationsterminus (ter); und
(iv) wobei die genomischen Positionen der phylogenetischen Markergene in einer vorberechneten linearen Skala von 0-100 in Bezug auf die Positionen des Replikationsursprungs (ori) und des Replikationsterminus (ter) der jeweiligen bakteriellen Genome, die die vorberechnete Referenzsequenzdatenbank bilden, dargestellt wurden,

und wobei der Schritt des Erstellens der vorberechneten Referenzsequenzdatenbank ferner das Erstellen einer genomischen Sequenzdatenbank umfasst, die ferner Sequenzen der anvisierten phylogenetischen Markergene von allen verfügbaren vollständig sequenzierten bakteriellen Genomen umfasst.

5. Prozessorimplementiertes Verfahren nach Anspruch 1, wobei die taxonomische Verteilung der Mehrzahl bakterieller Organismen, die aus der gesammelten Mikrobiomprobe identifiziert wurden, als die relative Häufigkeit von mindestens einem von (i) der gemessenen Leseabdeckung eines der phylogenetischen Markergene und (ii) einem Durchschnitt der gemessenen Leseabdeckung der zwei oder mehr phylogenetischen Markergene interpretiert wird.

6. Prozessorimplementiertes Verfahren nach Anspruch 1, wobei der Schritt des Anpassens einer linearen Funktion ferner mindestens eines von Folgendem umfasst:

Anpassen der linearen Funktion der Form y = $mx$ + c, wobei $m = \frac{y_B - y_A}{x_B - x_A}$ und $c = y_A - \frac{y_B - y_A}{x_B - x_A} \times x_A$,
wobei $y_A$ und $y_B$ die gemessene Leseabdeckung für zwei phylogenetische Markergene *A* bzw. *B* darstellen und $x_A$ und $x_B$ die entsprechenden genomischen Positionen der zwei phylogenetischen Markergene *A* bzw. *B* darstellen, und
Anpassen der linearen Funktion der Form y = $mx$ + c unter Verwendung einer linearen Regression, wobei gemessene Leseabdeckungen ($y_A$, $y_B$, $y_C$... $y_N$) für mehr als zwei phylogenetische Markergene *(A, B, C ...... N)* und entsprechende genomische Positionen ($x_A$, $x_B$, $x_C$... $x_N$) für mehr als zwei phylogenetische Markergene *(A, B, C ...... N)* berücksichtigt werden.

7. Prozessorimplementiertes Verfahren nach Anspruch 1, wobei die anvisierten phylogenetischen Markergene so ausgewählt werden, dass die effektiven Positionen der Markergene durch einen Abstand von >= 5 % des Abstands zwischen Replikationsursprungs- (ori) und Replikationsterminus- (ter) Positionen in einer Mehrheit der bakteriellen Organismen getrennt sind, von denen erwartet wird, dass sie in einer Umgebung vorhanden sind, aus der die Mikrobiomprobe gesammelt wurde, wobei das Berechnen einer effektiven Position eines phylogenetischen Markergens auf einem bakteriellen Genom das Berechnen eines Durchschnitts der Positionen für die eine oder die mehreren Kopien desselben phylogenetischen Markergens umfasst und das Berechnen einer effektiven Leseabdeckung eines phylogenetischen Markergens auf einem bakteriellen Genom das Berechnen einer durchschnittlichen Leseabdeckung für die eine oder die mehreren Kopien desselben phylogenetischen Markergens umfasst, wenn mehrere Kopien derselben phylogenetischen Markergene in einem beliebigen der Mehrzahl bakterieller Organismen vorhanden sind, die aus der gesammelten Mikrobiomprobe identifiziert wurden, und wobei die effektive Leseabdeckung und die effektive Position der zwei oder mehr phylogenetischen Markergene verwendet werden, um die lineare Gleichung der Form y = $mx$ + c.

8. Prozessorimplementiertes Verfahren nach Anspruch 1, wobei das Schätzen der erwarteten Leseabdeckung am Replikationsursprung $y_{ori}$ und einer erwarteten Leseabdeckung am Replikationsterminus $y_{ter}$ ferner das Erzeugen einer großen Verteilung von Verhältnissen der Leseabdeckung bei 16S-rRNA in Bezug auf die Leseabdeckung am Replikationsterminus (ter) für eine Mehrzahl von Bakterien aus vorbestehenden sequenzierten Daten der Gesamtgenom-Shotgun (WGS) und das Notieren des oberen 95· Perzentilwerts des Verhältnisses (T) umfasst, wobei dieser empirisch abgeleitete Wert von *T* verwendet wird, um sicherzustellen, dass geschätzte $y_{ori}$ und $y_{ter}$ auf die folgende Weise innerhalb biologisch machbarer Bereiche liegen,
wenn der geschätzte $y_{ter}$-Wert $<= \frac{y_{16s}}{T}$ ist, ein modifizierter Wert ( $y'_{ter}$ ) als $y'_{ter} = \frac{y_{16s}}{T}$ berechnet wird und ein modifizierter $y_{ori}$-Wert ( $y'_{ori}$ ) anschließend als $y'_{ori} = y'_{ter} - m \times 100$ berechnet wird und wobei $y_{16s}$ die effektive Abdeckung des 16S-rRNA-Markergens darstellt und wobei das Verhältnis T als ein Verhältnis der Leseabdeckung an einer beliebigen anderen ausgewählten genomischen Region in Bezug auf die Leseabdeckung am Replikationsterminus für die Mehrzahl von Bakterien aus vorbestehenden sequenzierten Daten der Gesamtgenom-Shotgun (WGS) berechnet werden kann.

9. System (100) zur gleichzeitigen Interpretation der taxonomischen Verteilung und der Replikationsraten von Mikroben, die mikrobielle Gemeinschaften bilden, wobei das System umfasst:

ein Probensammelmodul (102) zum Sammeln der Mikrobiomprobe aus einer gegebenen Umgebung;
ein DNA-Extraktionsmodul (104) zum Extrahieren bakterieller genomischer DNA aus einer Mehrzahl bakterieller Organismen, die die gesammelte Mikrobiomprobe bilden;

ein PCR-Amplifikationsmodul (106) und einen Sequenzer (108) zum Durchführen einer Amplikonsequenzierung an der extrahierten bakteriellen genomischen DNA, umfassend mindestens eines von (i) Anvisieren von zwei oder mehr phylogenetischen Markergenen und (ii) Auswählen eines Teils aus jedem der zwei oder mehr phylogenetischen Markergene, um eine Mehrzahl von DNA-Sequenzfragment-Reads zu erhalten, wobei die phylogenetischen Markergene in Genomen von Organismen gefunden werden und die zur Identifikation der taxonomischen Abstammung des Organismus verwendet werden;

einen Speicher (116);

und einen Prozessor (114) in Kommunikation mit dem Speicher (116), wobei der Prozessor konfiguriert ist, um die folgenden Schritte durchzuführen:

Abbilden der Mehrzahl der DNA-Sequenzfragment-Reads auf eine vorberechnete Referenzsequenzdatenbank einer Mehrzahl verfügbarer vollständig sequenzierter bakterieller Genome;

Identifizieren einer Mehrzahl bakterieller Organismen in der gesammelten Mikrobiomprobe und Zuweisen einer taxonomischen Klassifikation zu der identifizierten Mehrzahl bakterieller Organismen basierend auf dem Abbilden der Mehrzahl der DNA-Sequenzfragment-Reads auf die vorberechnete Referenzsequenzdatenbank;

Messen der Leseabdeckung an den genomischen Positionen der zwei oder mehr phylogenetischen Markergene für die Mehrzahl identifizierter bakterieller Organismen basierend auf dem Abbilden der Mehrzahl der DNA-Sequenzfragment-Reads auf die vorberechnete Referenzsequenzdatenbank, und wobei die gemessene Leseabdeckung für die Interpretation der taxonomischen Verteilung der Mehrzahl bakterieller Organismen verwendet wird, die aus der gesammelten Mikrobiomprobe identifiziert wurden;

Anpassen einer linearen Funktion der Form y = $mx$ + c für jeden der Mehrzahl bakterieller Organismen unter Verwendung der gemessenen Leseabdeckung und der Informationen der genomischen Positionen, die den zwei oder mehr phylogenetischen Markergenen entsprechen, in Bezug auf einen Replikationsursprung (ori) und einen Replikationsterminus (ter), die für jeden der Mehrzahl bakterieller Organismen spezifisch sind, die aus der gesammelten Mikrobiomprobe identifiziert wurden;

Erhalten einer Steigung *(m)* aus der angepassten linearen Funktion der Form y = $mx$ + c für jeden der Mehrzahl bakterieller Organismen, die aus der gesammelten Mikrobiomprobe identifiziert wurden;

Schätzen, für jeden der Mehrzahl bakterieller Organismen, die aus der gesammelten Mikrobiomprobe identifiziert wurden, einer erwarteten Leseabdeckung am Replikationsursprung $y_{ori}$ unter Verwendung der erhaltenen Steigung ($m$) und des Werts c aus der angepassten linearen Funktion;

Schätzen, für jeden der Mehrzahl bakterieller Organismen, die aus der gesammelten Mikrobiomprobe identifiziert wurden, einer erwarteten Leseabdeckung am Replikationsterminus $y_{ter}$ unter Verwendung der erhaltenen Steigung ($m$) und des Werts c aus der angepassten linearen Funktion; und

Interpretieren, für jeden der Mehrzahl bakterieller Organismen, die aus der gesammelten Mikrobiomprobe identifiziert wurden, einer Replikationsrate unter Verwendung von mindestens einem von (i) Steigungen *(m)* und (ii) einem Verhältnis von $\dfrac{y_{ori}}{y_{ter}}$ .

10. System nach Anspruch 9, wobei die zwei oder mehr phylogenetischen Markergene 16S rRNA, CPN60, 5S rRNA, gyrB, rpoB und tufA umfassen, und wobei die vorberechnete Referenzsequenzdatenbank den Abstand der phylogenetischen Markergene vom Replikationsursprung (ori) und dem Replikationsterminus (ter) eines kreisförmigen Chromosoms von allen verfügbaren vollständig sequenzierten bakteriellen Genomen umfasst.

11. System nach Anspruch 10, wobei die erstellte vorberechnete Referenzsequenzdatenbank ferner Folgendes umfasst:

(i) Ermitteln der genomischen Position der phylogenetischen Markergene für alle verfügbaren vollständig sequenzierten bakteriellen Genome;

(ii) Erfassen einer historischen genomischen Position des Replikationsursprungs (ori) und des Replikationsterminus (ter) für alle verfügbaren vollständig sequenzierten bakteriellen Genome;

(iii) Erstellen der genomischen Positionsdatenbank der phylogenetischen Markergene in Bezug auf den Abstand vom Replikationsursprung (ori) und dem Replikationsterminus (ter); und

(iv) wobei die genomischen Positionen der phylogenetischen Markergene in einer vorberechneten linearen Skala von 0-100 in Bezug auf die Positionen des Replikationsursprungs (ori) und des Replikationsterminus (ter) der jeweiligen bakteriellen Genome, die die vorberechnete Referenzsequenzdatenbank bilden, dargestellt wurden,

und wobei der Schritt des Erstellens der vorberechneten Referenzsequenzdatenbank ferner das Erstellen einer genomischen Sequenzdatenbank umfasst, die ferner Sequenzen der anvisierten phylogenetischen Markergene von allen verfügbaren vollständig sequenzierten bakteriellen Genomen umfasst.

12. System nach Anspruch 9, wobei der Schritt des Anpassens einer linearen Funktion ferner mindestens eines von Folgendem umfasst:

Anpassen der linearen Funktion der Form y = mx + c, wobei $m = \frac{y_B - y_A}{x_B - x_A}$ und $c = y_A - \frac{y_B - y_A}{x_B - x_A} \times x_A$, wobei $y_A$ und $y_B$ die gemessene Leseabdeckung für zwei phylogenetische Markergene A bzw. B darstellen und $x_A$ und $x_B$ die entsprechenden genomischen Positionen der zwei phylogenetischen Markergene A bzw. B darstellen; und

Anpassen der linearen Funktion der Form y = mx + c unter Verwendung einer linearen Regression, wobei gemessene Leseabdeckungen ($y_A$, $y_B$, $y_C$... $y_N$) für mehr als zwei phylogenetische Markergene (A, B, C ...... N) und entsprechende genomische Positionen ($x_A$, $x_B$, $x_C$... $x_n$) für mehr als zwei phylogenetische Markergene (A, B, C ...... N) berücksichtigt werden.

13. System nach Anspruch 9, wobei die anvisierten phylogenetischen Markergene so ausgewählt sind, dass die effektiven Positionen der Markergene durch einen Abstand >=5 % des Abstands zwischen Replikationsursprungs- (ori) und Replikationsterminus- (ter) Positionen in einer Mehrheit der bakteriellen Organismen getrennt sind, von denen erwartet wird, dass sie in einer Umgebung vorhanden sind, aus der die Mikrobiomprobe entnommen wurde, wobei das Berechnen einer effektiven Position eines phylogenetischen Markergens auf einem bakteriellen Genom das Berechnen eines Durchschnitts der Positionen für die eine oder die mehreren Kopien desselben phylogenetischen Markergens umfasst und das Berechnen einer effektiven Leseabdeckung eines phylogenetischen Markergens auf einem bakteriellen Genom das Berechnen einer durchschnittlichen Leseabdeckung für die eine oder die mehreren Kopien desselben phylogenetischen Markergens umfasst, wenn mehrere Kopien derselben phylogenetischen Markergene in einem beliebigen der Mehrzahl von bakteriellen Organismen vorhanden sind, die aus der entnommenen Mikrobiomprobe identifiziert wurden, und wobei die effektive Leseabdeckung und die effektive Position der zwei oder mehr phylogenetischen Markergene verwendet werden, um der linearen Gleichung der Form y = mx + c zu entsprechen.

14. Ein oder mehrere nichtflüchtige maschinenlesbare Informationsspeichermedien, die eine oder mehrere Anweisungen umfassen, die bei Ausführung durch einen oder mehrere Hardwareprozessoren Folgendes bewirken:

Sammeln einer Mikrobiomprobe aus einer gegebenen Umgebung (202);
Extrahieren bakterieller genomischer DNA (Desoxyribonukleinsäure) aus einer Mehrzahl bakterieller Organismen, die die gesammelte Mikrobiomprobe (204) bilden;
Durchführen einer Amplikonsequenzierung durch ein PCR (Polymerasekettenreaktion)-Amplifikationsmodul (106) und einen Sequenzer (108) an der extrahierten bakteriellen genomischen DNA, umfassend mindestens eines von (i) Anvisieren von zwei oder mehr phylogenetischen Markergenen und (ii) Auswählen eines Teils aus jedem der zwei oder mehr phylogenetischen Markergene, um eine Mehrzahl von DNA-Sequenzfragment-Reads zu erhalten, wobei die zwei oder mehr phylogenetischen Markergene in Genomen von Organismen gefunden werden und die zur Identifikation der taxonomischen Abstammung des Organismus (206) verwendet werden;
Abbilden der Mehrzahl der DNA-Sequenzfragment-Reads auf eine vorberechnete Referenzsequenzdatenbank einer Mehrzahl verfügbarer vollständig sequenzierter bakterieller Genome (208);
Identifizieren einer Mehrzahl bakterieller Organismen in der gesammelten Mikrobiomprobe und Zuweisen einer taxonomischen Klassifikation zu der identifizierten Mehrzahl bakterieller Organismen basierend auf dem Abbilden der Mehrzahl der DNA-Sequenzfragment-Reads auf die vorberechnete Referenzsequenzdatenbank (210);
Messen der Leseabdeckung an den genomischen Positionen der zwei oder mehr phylogenetischen Markergene für die Mehrzahl identifizierter bakterieller Organismen basierend auf dem Abbilden der Mehrzahl der DNA-Sequenzfragment-Reads auf die vorberechnete Referenzsequenzdatenbank, und wobei die gemessene Leseabdeckung für die Interpretation der taxonomischen Verteilung der Mehrzahl bakterieller Organismen verwendet wird, die aus der gesammelten Mikrobiomprobe (212) identifiziert wurden;
Anpassen einer linearen Funktion der Form y = mx + c für jeden der Mehrzahl bakterieller Organismen unter Verwendung der gemessenen Leseabdeckung und der Informationen der genomischen Positionen, die den zwei oder mehr phylogenetischen Markergenen entsprechen, in Bezug auf einen Replikationsursprung (ori) und einen Replikationsterminus (ter), die für jeden der Mehrzahl bakterieller Organismen spezifisch sind, die aus der gesammelten Mikrobiomprobe (214) identifiziert wurden;

Erhalten einer Steigung *(m)* aus der angepassten linearen Funktion der Form *y = mx* + c für jeden der Mehrzahl bakterieller Organismen, die aus der gesammelten Mikrobiomprobe (216) identifiziert wurden;

Schätzen, für jeden der Mehrzahl bakterieller Organismen, die aus der gesammelten Mikrobiomprobe identifiziert wurden, einer erwarteten Leseabdeckung am Replikationsursprung $y_{ori}$ unter Verwendung der erhaltenen Steigung *(m)* und des Werts c aus der angepassten linearen Funktion (218);

Schätzen, für jeden der Mehrzahl bakterieller Organismen, die aus der gesammelten Mikrobiomprobe identifiziert wurden, einer erwarteten Leseabdeckung am Replikationsterminus $y_{ter}$ unter Verwendung der erhaltenen Steigung *(m)* und des Werts c aus der angepassten linearen Funktion (220); und

Interpretieren, für jeden der Mehrzahl bakterieller Organismen, die aus der gesammelten Mikrobiomprobe identifiziert wurden, einer Replikationsrate unter Verwendung von mindestens einem von (i) Steigungen *(m)* und (ii) einem Verhältnis von $\dfrac{y_{ori}}{y_{ter}}$ (222).

**15.** Ein oder mehrere nichtflüchtige maschinenlesbare Informationsspeichermedien nach Anspruch 14, wobei der Schritt des Anpassens einer linearen Funktion mindestens eines von Folgendem umfasst:

$$m = \frac{y_B - y_A}{x_B - x_A} \quad \text{und} \quad c = y_A - \frac{y_B - y_A}{x_B - x_A} \times x_A \,,$$

Anpassen der linearen Funktion der Form y = *mx* + c, wobei und wobei $y_A$ und $y_B$ die gemessene Leseabdeckung für zwei phylogenetische Markergene *A* bzw. *B* darstellen und $x_A$ und $x_B$ die entsprechenden genomischen Positionen der zwei phylogenetischen Markergene *A* bzw. *B* darstellen, und

Anpassen der linearen Funktion der Form y = *mx* + c unter Verwendung einer linearen Regression, wobei gemessene Leseabdeckungen ($y_A$, $y_B$, $y_C$... $y_N$) für mehr als zwei phylogenetische Markergene *(A, B, C ...... N)* und entsprechende genomische Positionen ($x_A$, $x_B$, $x_C$... $x_N$) für mehr als zwei phylogenetische Markergene *(A, B, C ...... N)* berücksichtigt werden.

**Revendications**

**1.** Procédé (200) d'interprétation simultanée de la distribution taxonomique et des taux de réplication de microbes constituant des communautés microbiennes, le procédé comprenant :

la collecte d'un échantillon de microbiome à partir d'un environnement donné (202) ;

l'extraction d'ADN génomique bactérien (acide désoxyribonucléique) à partir d'une pluralité d'organismes bactériens constituant l'échantillon de microbiome collecté (204) ;

la réalisation d'un séquençage d'amplicons par un module d'amplification PCR (réaction en chaîne par polymérase) (106) et un séquenceur (108), sur l'ADN génomique bactérien extrait comprenant au moins l'un parmi (i) le ciblage de deux gènes marqueurs phylogénétiques ou plus et (ii) la sélection d'une partie à partir de chacun des deux gènes marqueurs phylogénétiques ou plus pour obtenir une pluralité de lectures de fragment de séquence d'ADN, dans lequel les deux gènes marqueurs phylogénétiques ou plus sont trouvés dans des génomes d'organismes, et qui sont utilisés pour l'identification de la lignée taxonomique de l'organisme (206) ;

le mappage, par un processeur (114), de la pluralité des lectures de fragment de séquence d'ADN avec une base de données de séquences de référence préétablie contenant une pluralité de génomes bactériens complètement séquencés disponibles (208) ;

l'identification, par le processeur (114), d'une pluralité d'organismes bactériens dans l'échantillon de microbiome collecté et l'attribution d'une classification taxonomique à la pluralité identifiée d'organismes bactériens sur la base du mappage de la pluralité des lectures de fragment de séquence d'ADN avec la base de données de séquences de référence préétablie (210) ;

la mesure, par le processeur (114), d'une couverture de lecture aux emplacements génomiques des deux gènes marqueurs phylogénétiques ou plus pour la pluralité d'organismes bactériens identifiés sur la base du mappage de la pluralité des lectures de fragment de séquence d'ADN avec la base de données de séquences de référence préétablie, et dans lequel la couverture de lecture mesurée est utilisée pour l'interprétation de la distribution taxonomique de la pluralité d'organismes bactériens identifiés à partir de l'échantillon de microbiome collecté (212) ;

l'ajustement, par le processeur (114), d'une fonction linéaire de la forme *y = mx* + c pour chacun de la pluralité d'organismes bactériens en utilisant la couverture de lecture mesurée et les informations des emplacements génomiques correspondant aux deux gènes marqueurs phylogénétiques ou plus par rapport à une origine de réplication (ori) et à une extrémité de réplication (ter) spécifiques à chacun de la pluralité d'organismes bactériens identifiés à partir de l'échantillon de microbiome collecté (214) ;

l'obtention, par le processeur (114), d'une pente ($m$) à partir de la fonction linéaire ajustée de la forme $y = mx + c$ pour chacun de la pluralité d'organismes bactériens identifiés à partir de l'échantillon de microbiome collecté (216) ;

l'estimation, par le processeur (114), pour chacun de la pluralité d'organismes bactériens identifiés à partir de l'échantillon de microbiome collecté d'une couverture de lecture attendue à l'origine de réplication $y_{ori}$ en utilisant la pente *(m)* obtenue et la valeur c à partir de la fonction linéaire ajustée (218) ;

l'estimation, par le processeur (114), pour chacun de la pluralité d'organismes bactériens identifiés à partir de l'échantillon de microbiome collecté d'une couverture de lecture attendue à l'extrémité de réplication $y_{ter}$ en utilisant la pente *(m)* obtenue et la valeur c à partir de la fonction linéaire ajustée (220) ; et

l'interprétation, par un processeur (114), pour chacun de la pluralité d'organismes bactériens identifiés à partir de l'échantillon de microbiome collecté d'un taux de réplication en utilisant au moins l'un de (i) pentes *(m)* et (ii) ratio

$$\frac{y_{ori}}{y_{ter}}$$ (222).

2. Procédé mis en œuvre par processeur selon la revendication 1, dans lequel l'environnement donné pour collecter l'échantillon de microbiome comprend :

(i) la collecte de l'échantillon de microbiome à partir de l'un des sites corporels humain comprenant l'intestin, la peau, les cheveux, le nasopharynx et à partir de fluides corporels comprenant la salive, l'urine, le sang, les selles, les expectorations et le cérumen ;

(ii) la collecte de l'échantillon de microbiome à partir de l'un des sites corporels de l'animal comprenant l'intestin, la peau, les cheveux, le nasopharynx et à partir de fluides corporels comprenant la salive, l'urine, le sang, les selles, les expectorations et le cérumen ;

(iii) la collecte de l'échantillon de microbiome à partir de différentes parties d'une plante, à savoir, l'endosphère, la rhizosphère, le rhizoplane, la feuille, le fruit, la graine, ainsi que les extraits de plantes et de produits dérivés de plantes ;

(iv) la collecte de l'échantillon de microbiome à partir de sources environnementales comprenant des eaux usées, un bioréacteur, un lit de rivière, un lit océanique et l'air ; et

(v) la collecte de l'échantillon de microbiome à partir de matière biologique ou organique stockée, comprenant des aliments crus, des aliments transformés, des graines alimentaires, des médicaments issus de produits naturels et des formulations probiotiques destinées à un usage thérapeutique.

3. Procédé mis en œuvre par processeur selon la revendication 1, dans lequel les deux gènes marqueurs phylogénétiques ou plus comprennent 16S rRNA, CPN60, 5S rRNA, gyrB, rpoB et tufA, et dans lequel la base de données de séquences de référence préétablie comprend la distance des gènes marqueurs phylogénétiques à partir de l'origine de réplication (ori) et de l'extrémité de réplication (ter) d'un chromosome circulaire à partir de tous les génomes bactériens complètement séquencés disponibles.

4. Procédé mis en œuvre par processeur selon la revendication 3, dans lequel la base de données de séquences de référence préétablie créée comprend en outre :

(i) la détermination de l'emplacement génomique des gènes marqueurs phylogénétiques pour tous les génomes bactériens complètement séquencés disponibles ;

(ii) l'acquisition de l'emplacement génomique historique de l'origine de réplication (ori) et de l'extrémité de réplication (ter) pour tous les génomes bactériens complètement séquencés disponibles ;

(iii) la création de la base de données d'emplacements génomiques des gènes marqueurs phylogénétiques en termes de distance à partir de l'origine de réplication (ori) et de l'extrémité de réplication (ter) ; et

(iv) les emplacements génomiques des gènes marqueurs phylogénétiques ont été représentés sur une échelle linéaire préétablie de 0 à 100 par rapport aux emplacements de l'origine de réplication (ori) et de l'extrémité de réplication (ter) des génomes bactériens respectifs constituant la base de données de séquences de référence préétablie,

et dans lequel l'étape de création de la base de données de séquences de référence préétablie comprend en outre la création d'une base de données de séquences génomiques qui comprend en outre des séquences des gènes marqueurs phylogénétiques ciblés à partir de tous les génomes bactériens complètement séquencés disponibles.

**5.** Procédé mis en œuvre par processeur selon la revendication 1, dans lequel la distribution taxonomique de la pluralité d'organismes bactériens identifiés à partir de l'échantillon de microbiome collecté est interprétée comme l'abondance relative d'au moins l'un de (i) une couverture de lecture mesurée de l'un des gènes marqueurs phylogénétiques, et (ii) une moyenne de la couverture de lecture mesurée des deux gènes marqueurs phylogénétiques ou plus.

**6.** Procédé mis en œuvre par processeur selon la revendication 1, dans lequel l'étape d'ajustement d'une fonction linéaire comprend en outre au moins l'un de :

l'ajustement de la fonction linéaire de la forme y = $mx$ + c dans laquelle $m = \frac{y_B - y_A}{x_B - x_A}$ et c = $y_A$ - $\frac{y_B - y_A}{x_B - x_A} \times x_A$ , dans laquelle $y_A$ et $y_B$ représentent la couverture de lecture mesurée pour deux gènes marqueurs phylogénétiques A et B respectivement, et $x_A$ et $x_B$ représentent les emplacements génomiques correspondants des deux gènes marqueurs phylogénétiques A et B respectivement, et

l'ajustement de la fonction linéaire de la forme y = $mx$ + c en utilisant une régression linéaire dans laquelle des couvertures de lecture mesurées ($y_A$, $y_B$, $y_C$... $Y_N$) pour plus de deux gènes marqueurs phylogénétiques (A, B, C ...... N) et des emplacements génomiques correspondants ($x_A$, $x_B$, $x_C$... $x_N$) pour plus de deux gènes marqueurs phylogénétiques (A, B, C ...... N) sont considérés.

**7.** Procédé mis en œuvre par processeur selon la revendication 1, dans lequel les gènes marqueurs phylogénétiques ciblés sont sélectionnés de telle sorte que les emplacements effectifs des gènes marqueurs sont séparés par une distance >= 5 % de la distance entre des emplacements d'origine de réplication (ori) et d'extrémité de réplication (ter) dans une majorité des organismes bactériens qui sont censés être présents dans un environnement à partir duquel l'échantillon de microbiome a été collecté, conformément aux preuves issues de la littérature, dans lequel le calcul d'un emplacement effectif d'un gène marqueur phylogénétique sur un génome bactérien comprend le calcul d'une moyenne des emplacements pour les une ou plusieurs copies du même gène marqueur phylogénétique, et le calcul d'une couverture de lecture effective d'un gène marqueur phylogénétique sur un génome bactérien comprend le calcul d'une couverture de lecture moyenne pour les une ou plusieurs copies du même gène marqueur phylogénétique, lorsque de multiples copies des mêmes gènes marqueurs phylogénétiques sont présents dans l'un quelconque de la pluralité d'organismes bactériens identifiés à partir de l'échantillon de microbiome collecté, et dans lequel la couverture de lecture effective et l'emplacement effectif des deux gènes marqueurs phylogénétiques ou plus sont utilisés pour ajuster l'équation linéaire de la forme y = $mx$ + c.

**8.** Procédé mis en œuvre par processeur selon la revendication 1, dans lequel l'estimation de la couverture de lecture attendue à l'origine de réplication $y_{ori}$ et d'une couverture de lecture attendue à l'extrémité de réplication $y_{ter}$ comprend en outre la génération d'une large distribution de ratios de couverture de lecture au niveau du 16S rRNA par rapport à la couverture de lecture à l'extrémité de réplication (ter) pour une pluralité de bactéries à partir de données de séquençage génomique complet par shotgun (WGS) préexistantes et la notation de la valeur du 95e centile supérieur du ratio (T), dans lequel cette valeur empiriquement dérivée de T est utilisée pour s'assurer que les valeurs estimées de $y_{ori}$ et $y_{ter}$ se situent dans des plages biologiquement plausibles de la manière suivante,

si la valeur estimée de $y_{ter}$ est $<= \frac{y_{16s}}{T}$ , une valeur modifiée ( $y'_{ter}$ ) est calculée comme $y'_{ter} = \frac{y_{16s}}{T}$ , et une valeur modifiée de $y_{ori}$ ( $y'_{ori}$ ) est ensuite calculée comme $y'_{ori} = y'_{ter} - m \times 100$ et dans lequel $y_{16s}$ représente la couverture efficace du gène marqueur 16S rRNA, et dans lequel le ratio T peut être calculé comme un ratio de couverture de lecture à toute autre région génomique sélectionnée par rapport à la couverture de lecture à l'extrémité de réplication pour la pluralité de bactéries à partir de données de séquençage génomique complet par shotgun (WGS) préexistantes.

**9.** Système (100) d'interprétation simultanée de la distribution taxonomique et des taux de réplication de microbes constituant des communautés microbiennes, le système comprenant :

un module de collecte d'échantillon (102) pour collecter l'échantillon de microbiome à partir d'un environnement donné ;
un module d'extraction d'ADN (104) pour extraire de l'ADN génomique bactérien à partir d'une pluralité d'organismes bactériens constituant l'échantillon de microbiome collecté ;
un module d'amplification par PCR (106) et un séquenceur (108) pour réaliser un séquençage d'amplicons, sur l'ADN génomique bactérien extrait comprenant au moins l'un parmi (i) le ciblage de deux gènes marqueurs phylogénétiques ou plus et (ii) la sélection d'une portion à partir de chacun des deux gènes marqueurs phylogénétiques ou plus pour obtenir une pluralité de lectures de fragment de séquence d'ADN, dans lequel les gènes marqueurs phylogénétiques sont trouvés dans des génomes d'organismes, et qui sont utilisés pour l'identification de la lignée taxonomique de l'organisme ;

une mémoire (116) ;

et un processeur (114) en communication avec la mémoire (116), dans lequel le processeur est configuré pour réaliser les étapes de :

le mappage de la pluralité des lectures de fragment de séquence d'ADN avec une base de données de séquences de référence préétablie contenant une pluralité de génomes bactériens complètement séquencés disponibles ;

l'identification d'une pluralité d'organismes bactériens dans l'échantillon de microbiome collecté et l'attribution d'une classification taxonomique à la pluralité identifiée d'organismes bactériens sur la base du mappage de la pluralité des lectures de fragment de séquence d'ADN avec la base de données de séquences de référence préétablie ;

la mesure d'une couverture de lecture aux emplacements génomiques des deux gènes marqueurs phylogénétiques ou plus pour la pluralité d'organismes bactériens identifiés sur la base du mappage de la pluralité des lectures de fragment de séquence d'ADN avec la base de données de séquences de référence préétablie, et dans lequel la couverture de lecture mesurée est utilisée pour l'interprétation de la distribution taxonomique de la pluralité d'organismes bactériens identifiés à partir de l'échantillon de microbiome collecté ;

l'ajustement d'une fonction linéaire de la forme $y = mx + c$ pour chacun de la pluralité d'organismes bactériens en utilisant la couverture de lecture mesurée et les informations des emplacements génomiques correspondant aux deux gènes marqueurs phylogénétiques ou plus par rapport à une origine de réplication (ori) et à une extrémité de réplication (ter) spécifiques à chacun de la pluralité d'organismes bactériens identifiés à partir de l'échantillon de microbiome collecté ;

l'obtention d'une pente *(m)* à partir de la fonction linéaire ajustée de la forme $y = mx + c$ pour chacun de la pluralité d'organismes bactériens identifiés à partir de l'échantillon de microbiome collecté ;

l'estimation pour chacun de la pluralité d'organismes bactériens identifiés à partir de l'échantillon de microbiome collecté d'une couve$^2$rture de lecture attendue à l'origine de réplication $y_{ori}$ en utilisant la pente *(m)* obtenue et la valeur c à partir de la fonction linéaire ajustée ;

l'estimation pour chacun de la pluralité d'organismes bactériens identifiés à partir de l'échantillon de microbiome collecté d'une couverture de lecture attendue à l'extrémité de réplication $y_{ter}$ en utilisant la pente *(m)* obtenue et la valeur c à partir de la fonction linéaire ajustée ; et

l'interprétation pour chacun de la pluralité d'organismes bactériens identifiés à partir de l'échantillon de microbiome collecté d'un taux de réplication en utilisant au moins l'un de (i) pentes *(m)* et (ii) ratio $\frac{y_{ori}}{y_{ter}}$.

10. Système selon la revendication 9, dans lequel les deux gènes marqueurs phylogénétiques ou plus comprennent 16S rRNA, CPN60, 5S rRNA, gyrB, rpoB et tufA, et dans lequel la base de données de séquences de référence préétablie comprend la distance des gènes marqueurs phylogénétiques à partir de l'origine de réplication (ori) et de l'extrémité de réplication (ter) d'un chromosome circulaire à partir de tous les génomes bactériens complètement séquencés disponibles.

11. Système selon la revendication 10, dans lequel la base de données de séquences de référence précalculées créée comprend en outre :

(v) la détermination de l'emplacement génomique des gènes marqueurs phylogénétiques pour tous les génomes bactériens complètement séquencés disponibles ;

(vi) l'acquisition de l'emplacement génomique historique de l'origine de réplication (ori) et de l'extrémité de réplication (ter) pour tous les génomes bactériens complètement séquencés disponibles ;

(vii) la création de la base de données d'emplacements génomiques des gènes marqueurs phylogénétiques en termes de distance à partir de l'origine de réplication (ori) et de l'extrémité de réplication (ter) ; et

(viii) les emplacements génomiques des gènes marqueurs phylogénétiques ont été représentés sur une échelle linéaire préétablie de 0 à 100 par rapport aux emplacements de l'origine de réplication (ori) et de l'extrémité de réplication (ter) des génomes bactériens respectifs constituant la base de données de séquences de référence préétablie,

et dans lequel l'étape de création de la base de données de séquences de référence préétablie comprend en outre la création d'une base de données de séquences génomiques qui comprend en outre des séquences des gènes marqueurs phylogénétiques ciblés à partir de tous les génomes bactériens complètement séquencés disponibles.

12. Système selon la revendication 9, dans lequel l'étape d'ajustement d'une fonction linéaire comprend en outre au moins l'un de :

l'ajustement de la fonction linéaire de la forme y = $mx$ + c dans laquelle $m = \frac{y_B - y_A}{x_B - x_A}$ et $c = y_A - \frac{y_B - y_A}{x_B - x_A} \times x_A$, dans laquelle $y_A$ et $y_B$ représentent la couverture de lecture mesurée pour deux gènes marqueurs phylogénétiques $A$ et $B$ respectivement, et $x_A$ et $x_B$ représentent les emplacements génomiques correspondants des deux gènes marqueurs phylogénétiques $A$ et $B$ respectivement, et

l'ajustement de la fonction linéaire de la forme y = $mx$ + c en utilisant une régression linéaire dans laquelle des couvertures de lecture mesurées ($y_A$, $y_B$, $y_C$... $y_N$) pour plus de deux gènes marqueurs phylogénétiques ($A$, $B$, $C$ ...... N) et des emplacements génomiques correspondants ($x_A$, $x_B$, $x_C$... $x_N$) pour plus de deux gènes marqueurs phylogénétiques ($A$, $B$, $C$ ... ... N) sont considérés.

13. Système selon la revendication 9, dans lequel les gènes marqueurs phylogénétiques ciblés sont sélectionnés de telle sorte que les emplacements effectifs des gènes marqueurs sont séparés par une distance >= 5 % de la distance entre des emplacements d'origine de réplication (ori) et d'extrémité de réplication (ter) dans une majorité des organismes bactériens qui sont censés être présents dans un environnement à partir duquel l'échantillon de microbiome a été collecté, conformément aux preuves issues de la littérature, dans lequel le calcul d'un emplacement effectif d'un gène marqueur phylogénétique sur un génome bactérien comprend le calcul d'une moyenne des emplacements pour les une ou plusieurs copies du même gène marqueur phylogénétique, et le calcul d'une couverture de lecture effective d'un gène marqueur phylogénétique sur un génome bactérien comprend le calcul d'une couverture de lecture moyenne pour les une ou plusieurs copies du même gène marqueur phylogénétique, lorsque de multiples copies des mêmes gènes marqueurs phylogénétiques sont présents dans l'un quelconque de la pluralité d'organismes bactériens identifiés à partir de l'échantillon de microbiome collecté, et dans lequel la couverture de lecture effective et l'emplacement effectif des deux gènes marqueurs phylogénétiques ou plus sont utilisés pour ajuster l'équation linéaire de la forme y = $mx$ + c.

14. Un ou plusieurs supports de stockage d'informations lisibles par machine non transitoires comprenant une ou plusieurs instructions qui, lorsqu'elles sont exécutées par un ou plusieurs processeurs matériels, amènent :

la collecte d'un échantillon de microbiome à partir d'un environnement donné (202) ;
l'extraction d'ADN génomique bactérien (acide désoxyribonucléique) à partir d'une pluralité d'organismes bactériens constituant l'échantillon de microbiome collecté (204) ;
la réalisation d'un séquençage d'amplicons par un module d'amplification PCR (réaction en chaîne par polymérase) (106) et un séquenceur (108), sur l'ADN génomique bactérien extrait comprenant au moins l'un parmi (i) le ciblage de deux gènes marqueurs phylogénétiques ou plus et (ii) la sélection d'une partie à partir de chacun des deux gènes marqueurs phylogénétiques ou plus pour obtenir une pluralité de lectures de fragment de séquence d'ADN, dans lequel les deux gènes marqueurs phylogénétiques ou plus sont trouvés dans des génomes d'organismes, et qui sont utilisés pour l'identification de la lignée taxonomique de l'organisme (206) ;
le mappage de la pluralité des lectures de fragment de séquence d'ADN avec une base de données de séquences de référence préétablie contenant une pluralité de génomes bactériens complètement séquencés disponibles (208) ;
l'identification d'une pluralité d'organismes bactériens dans l'échantillon de microbiome collecté et l'attribution d'une classification taxonomique à la pluralité identifiée d'organismes bactériens sur la base du mappage de la pluralité des lectures de fragment de séquence d'ADN avec la base de données de séquences de référence préétablie (210) ;
la mesure d'une couverture de lecture aux emplacements génomiques des deux gènes marqueurs phylogénétiques ou plus pour la pluralité d'organismes bactériens identifiés sur la base du mappage de la pluralité des lectures de fragment de séquence d'ADN avec la base de données de séquences de référence préétablie, et dans lequel la couverture de lecture mesurée est utilisée pour l'interprétation de la distribution taxonomique de la pluralité d'organismes bactériens identifiés à partir de l'échantillon de microbiome collecté (212) ;
l'ajustement d'une fonction linéaire de la forme y = $mx$ + c pour chacun de la pluralité d'organismes bactériens en utilisant la couverture de lecture mesuré et les informations des emplacements génomiques correspondant aux deux gènes marqueurs phylogénétiques ou plus par rapport à une origine de réplication (ori) et à une extrémité de réplication (ter) spécifiques à chacun de la pluralité d'organismes bactériens identifiés à partir de l'échantillon de microbiome collecté (214) ;

l'obtention d'une pente *(m)* à partir de la fonction linéaire ajustée de la forme *y = mx + c* pour chacun de la pluralité d'organismes bactériens identifiés à partir de l'échantillon de microbiome collecté (216) ;

l'estimation pour chacun de la pluralité d'organismes bactériens identifiés à partir de l'échantillon de microbiome collecté d'une couverture de lecture attendue à l'origine de réplication $y_{ori}$ en utilisant la pente *(m)* obtenue et la valeur c à partir de la fonction linéaire ajustée (218) ;

l'estimation pour chacun de la pluralité d'organismes bactériens identifiés à partir de l'échantillon de microbiome collecté d'une couverture de lecture attendue à l'extrémité de réplication $y_{ter}$ en utilisant la pente *(m)* obtenue et la valeur c à partir de la fonction linéaire ajustée (220) ; et

l'interprétation pour chacun de la pluralité d'organismes bactériens identifiés à partir de l'échantillon de microbiome collecté d'un taux de réplication en utilisant au moins l'un de (i) pentes *(m)* et (ii) ratio $\dfrac{y_{ori}}{y_{ter}}$ (222).

15. Un ou plusieurs supports de stockage d'informations lisibles par machine non transitoires selon la revendication 14, dans lequel l'étape d'ajustement d'une fonction linéaire comprend au moins l'un de :

l'ajustement de la fonction linéaire de la forme y = *mx* + c dans laquelle $m = \dfrac{y_B - y_A}{x_B - x_A}$ et $c = y_A - \dfrac{y_B - y_A}{x_B - x_A} \times x_A$,

dans laquelle $y_A$ et $y_B$ représentent la couverture de lecture mesurée pour deux gènes marqueurs phylogénétiques *A* et *B* respectivement, et $x_A$ et $x_B$ représentent les emplacements génomiques correspondants des deux gènes marqueurs phylogénétiques *A* et *B* respectivement, et

l'ajustement de la fonction linéaire de la forme y = *mx* + c en utilisant une régression linéaire dans laquelle des couvertures de lecture mesurées ($y_A$, $y_B$, $y_C$... $y_N$) pour plus de deux gènes marqueurs phylogénétiques (*A, B, C ...... N)* et des emplacements génomiques correspondants ($x_A$, $x_B$, $x_C$... $x_N$) pour plus de deux gènes marqueurs phylogénétiques (*A, B, C ...... N*) sont considérés.

FIG. 1

collecting a microbiome sample from a given environment — 202

extracting bacterial genomic DNA (Deoxyribonucleic Acid) from a plurality of bacterial organisms constituting the collected microbiome sample — 204

performing an amplicon sequencing by a PCR (Polymerase chain reaction) amplification module and a sequencer, on the extracted bacterial genomic DNA comprising at least one of (i) targeting two or more phylogenetic marker genes and (ii) selecting a portion from each of the two or more phylogenetic marker genes to obtain a plurality of DNA sequence fragment reads, wherein the two or more phylogenetic marker genes are found in genomes of organisms, and which are used for identification of the taxonomic lineage of the organism — 206

mapping the plurality of the DNA sequence fragment reads to a precomputed reference sequence database of a plurality of available completely sequenced bacterial genomes — 208

identifying a plurality of bacterial organisms in the collected microbiome sample and assigning a taxonomic classification to the identified plurality of bacterial organisms based on the mapping of the plurality of the DNA sequence fragment reads to the precomputed reference sequence database — 210

200

measuring read coverage at the genomic locations of the two or more phylogenetic marker genes for the plurality of identified bacterial organisms based on the mapping of the plurality of the DNA sequence fragment reads to the precomputed reference sequence database, and wherein the measured read coverage is used for the interpretation of the taxonomic distribution of the plurality of bacterial organisms identified from the collected microbiome sample — 212

FIG. 2A ( A )

EP 4 260 321 B1

(A)

fitting a linear function of the form $y = mx + c$ for each of the plurality of bacterial organisms by using the measured read coverage and the information of the genomic locations corresponding to the two or more phylogenetic marker genes with respect to an origin of replication (ori) and a terminus of replication (ter) specific to each of the plurality of bacterial organisms identified from the collected microbiome sample — 214

obtaining a slope ($m$) from the fitted linear function of the form $y = mx + c$ for each of the plurality of bacterial organisms identified from the collected microbiome sample — 216

estimating for each of the plurality of bacterial organisms identified from the collected microbiome sample an expected read coverage at the origin of replication $y_{ori}$ using the slope ($m$) obtained and the value $c$ from the fitted linear function — 218

estimating for each of the plurality of bacterial organisms identified from the collected microbiome sample an expected read coverage at the terminus of replication $y_{ter}$ using the slope ($m$) obtained and the value $c$ from the fitted linear function — 220

interpreting for each of the plurality of bacterial organisms identified from the collected microbiome sample a replication rate using at least one of (i) slopes ($m$) and (ii) ratio of $\frac{y_{ori}}{y_{ter}}$ — 222

200

FIG. 2B

**Bacterial chromosomal replication process**

FIG. 3

FIG. 4

Distance from Ori

Ori

gene A

replication forks

gene A

gene B

Distance from Ori

No. of mapped genes (Y) or coverage

$$y - y_A = \frac{y_B - y_A}{x_B - x_A} * (x - x_A)$$

Ori

$y_{Ori}$

A $(x_A, y_A)$

B $(x_B, y_B)$

ter

$y_{ter}$

Ori (x=0)

$x_A$

$x_B$

$x_{ter}$

**Distance from Ori (X)**

Known parameters:  $x_{Ori}$,  $x_{ter}$,  $x_A$,  $x_B$,  $y_A$,  $y_B$

Parameters to derive:  $y_{Ori}$,  $y_{ter}$,  $\dfrac{y_B - y_A}{x_B - x_A}$  ← slope

$$\begin{Bmatrix} y_{Ori} = y_{peak} \\ y_{ter} = y_{trough} \end{Bmatrix}$$

FIG. 5

FIG. 6

FIG. 7A

FIG. 7B

FIG. 7C

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **KOREM et al.** *Growth dynamics of gut microbiota in health and disease inferred from single metagenomic samples*

- **BROWN et al.** *Measurement of bacterial replication rates in microbial communities*, https://doi.org/10.1038/nbt.3704